# EUROPEAN PATENT SPECIFICATION

(11) **EP 3 160 996 B1**
(45) Date of publication and mention of the grant of the patent: **08.06.2022**
(21) Application number: 15732165.4
(22) Date of filing: 26.06.2015
(51) Int. Cl.: C07K 16/08

(54) **ANTI-TNFA ANTIBODIES WITH PH-DEPENDENT ANTIGEN BINDING**
ANTI-TNFA-ANTIKÖRPER MIT PH-ABHÄNGIGER ANTIGENBINDUNG
ANTICORPS ANTI-TNFA À LIAISON ANTIGÉNIQUE SENSIBLE AU PH

(30) Priority: 30.06.2014 EP 14002231
(43) Date of publication of application: 03.05.2017
(73) Proprietor: Merck Patent GmbH, 64293 Darmstadt (DE)
(72) Inventor: GUENTHER, Ralf, 64347 Griesheim (DE); BECKER, Stefan, 64354 Reinheim (DE); RHIEL, Laura, 60385 Frankfurt am Main (DE); HOCK, Bjoern, 63477 Maintal (DE); SCHROETER, Christian, 64646 Heppenheim (DE)
(86) International application number: PCT/EP2015/001296
(87) International publication number: WO 2016/000813

(56) References cited:
- EP-A1- 2 275 443
- EP-A1- 2 762 564
- WO-A1-2013/046722
- WO-A2-2011/111007
- WO-A2-2012/065072
- US-A1- 2013 247 234
- ITO W ET AL: "The His-probe method: effects of histidine residues introduced into the complementary-determining regions of antibodies on antigen-antibody interactions at different pH values", FEBS LETTERS, ELSEVIER, AMSTERDAM, NL, vol. 309, no. 1, 1 August 1992 (1992-08-01), pages 85-88, XP008123385, ISSN: 0014-5793
- Tomoyuki Igawa ET AL: "Engineered Monoclonal Antibody with Novel Antigen-Sweeping Activity In Vivo", PLoS ONE, vol. 8, no. 5, 7 May 2013 (2013-05-07), page e63236, XP055172504, DOI: 10.1371/journal.pone.0063236
- Siva Charan Devanaboyina ET AL: "The effect of pH dependence of antibody-antigen interactions on subcellular trafficking dynamics", mAbs, vol. 5, no. 6, 1 November 2013 (2013-11-01), pages 851-859, XP055173157, ISSN: 1942-0862, DOI: 10.4161/mabs.26389

## Description

### Field of the Invention

The invention relates to anti-TNFa antibodies which are engineered to exhibit a pH-sensitive antigen binding. The invention is directed to biologically active variants of anti-TNFa antibody adalimumab (Humira^{®}) and fragments thereof, wherein the original adalimumab antibody or variant or fragment thereof is engineered by modifications of amino acid sequence within the variable regions. Specifically, the invention relates to biologically active variants of adalimumab or fragments thereof, wherein the CDR domains are modified by replacing amino acid residues by histidine residues.

The resulting modified anti-TNFa antibodies elicit improved pharmacokinetic properties with improved antigen-mediated IgG clearance and extended over-all serum half life.

### Background of the Invention

It is believed that therapeutic antibodies (mAbs) at least offer important treatment options for many diseases like inflammatory, autoimmune or oncological disorders. In 2012 there were 40 FDA-approved mAbs on the US market against various targets in oncology and anti-inflammatory disorders with ~ 38.5% share within the biologics market. Sales of ~$24.6 billion manifest the role of therapeutic antibodies as highest earning category of all biologics (Aggarwal, 2009, Aggarwal, 2014).

For therapeutic antibodies different biological outcomes are determined by the interaction profiles with four classes of naturally occurring interaction partners: antigen, neonatal Fc-receptor (FcRn), Fc-receptors (FcγRs), and factors of the complement system (Chan and Carter, 2010). Several strategies have been reported to optimize antibodies that aim for additional or improved functions and specificities. (Beck et al, 2010). Within antibodies there are two structural features that can be addressed for engineering. First, the variable fragment (Fv) that mediates interaction with the antigen, second the constant fragment (Fc) that is involved in antibody recycling or mediates interactions with immune cells.

For different antigens (e.g. cytokines and growth factors) there are multiple mechanisms of action, e.g. blocking of soluble ligands thereby preventing the interaction to its corresponding receptor or blocking of the receptor itself. (Chan and Carter, 2010). A lot of effort has been invested on improving functions towards Fv-engineering that is often valued by increasing specificity and/or binding affinity to respective antigens (Beck et al, 2010). One strategy to elevate antibody efficacy is to enhance the Fv - antigen interaction by affinity maturation approaches. Herein the use of display technologies for screening molecule libraries allows isolation of variants that exhibit superior affinity.

The constant fragment (Fc) and its linked properties can be modulated with altered outcomes for immunity or antibody recycling. Prominent examples for altered Fc-mediated immune functions are enhanced antibody-dependent cellular cytotoxicity (ADCC) and complementdependent cytotoxicity (CDC) that have been addressed to enhance antibody efficacy and to reduce the dosages.

Further strategies have been explored including direct and indirect arming of antibodies or modulation of specificities within multivalent antibodies (Carter 2011).

One important aspect of Fc-function corresponds to its critical role in antibody recycling that determines the long serum half life of human immunoglobulin 1 (IgG1). After cellular absorbtion via fluid phase pinocytosis, the Fc-portion of an IgG1 interacts in a pH-dependent manner with the neonatal Fc-receptor (FcRn) that leads to antibody capture in the acidified endosome (Kuo and Aveson, 2011). From there, antibodies are recycled back to the circulation and therefore can be protected from intracellular catabolism. Different mutational Fc-species with enhanced FcRn binding affinity were generated and tested for increased recycling rates with up to 4-fold extended serum half-life in cynomolgus studies by substituting three amino acids (Dall'Acqua et al, 2006).

Although most antibodies demonstrate highly efficient antigen blocking, there are drawbacks that are not fully addressed in the development process of therapeutic antibodies:
➢ In many therapeutic antibodies the antigen-binding sites bind to only one antigen molecule during the antibody's lifetime in plasma (Igawa et al, 2010).
➢ The dosing and frequency of antibody injections depends on the antigen synthesis rate between two injections as antigens are usually produced continuously *in vivo* (Igawa et al, 2010).
➢ High production costs & administration of large quantities of antibodies:
   Tocilicumab therapy: 8 mg/kg/month by i.v. (Maini et al, 2006), Adalimumab therapy: 40 mg every other week ($19,272 / per person / per year (Schabert et al. 2013).
➢ When antibodies bind soluble targets "antibody buffering" effects can occur in which the degradation of the antigen is prevented while being bound to the antibody. A pool of free antigen establishes from reversible dissociation of the antigen-antibody complex and therefore prolongs the *in vivo* persistence (O'Hear and Foote, 2005) / Finkelman et al, 1993)

Considering these drawbacks of therapeutic antibodies, there is a need for more efficient molecules that produce therapeutic responses without high dosing and/or frequent administration (Chapparo-Riggers et al, 2012).

One possibility to achieve these goals is the specific engineering of the variable and optionally the constant region of a new or well established and approved antibody. One approach is to develop antibodies that exhibit pH-sensitive antigen binding. It has been shown that rational or combinatorial incorporation of histidines in the binding interfaces of antibodies and other proteins (Sarkar et al., 2002, Chaparro-Riggers et al., 2012, Ito et al., 1992, Igawa et al., 2010, Igawa et al., 2013, Murtaugh et al., 2011, Gera et al., 2012) can be commonly used to engineer pH-dependent binding. The basis for the pH-sensitive binding arises from the histidine's sensitivity to get protonated as a result of lowered pH-values in the microenvironment. More in detail, the histidines need to undergo a pKa-change upon binding in order to get protonated in a physiological pH-range (Murtaugh et al., 2011). Protonation of histidine side chains in binding-interfaces can alter electrostatic interactions or may induce conformational changes that lead to pH-dependent differences in binding affinity (Gera et al., 2012). Balanced electrostatic and non-electrostatic components of the binding equilibrium determine the sensitivity of binding (Murtaugh et al., 2011).

Incorporation of pH-sensitivity into the antigen binding site can increase the number of antigen-binding cycles. Herein, pH-dependent antibodies bind with similar high or reduced sufficient affinity to their antigens at plasma pH (pH 7.4) and show decreased binding at acidic pH (pH 6) (Chaparro-Riggers et al., 2012, Igawa et al, 2010) resulting in a faster and increased dissociation of the antibody from its antigen binding site within the acidic endosome, thereby enabling recycling back to the plasma and reducing antigen-mediated clearance.

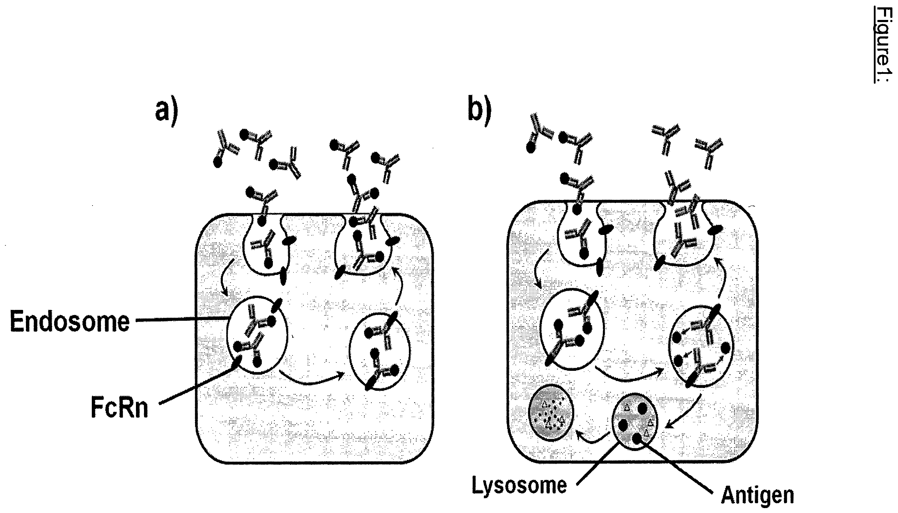

During the FcRn-mediated recycling (Fig.1a) of conventional antibodies that bind soluble targets, the antibody-antigen complex is recycled back to the extracellular space through the endosomal trafficking pathway (Roopenian and Akilesh, 2007). In contrast, pH-sensitive antibodies (Fig.1b) release their antigen from the antibody-antigen complex during the endosomal acidification (pH <6.5) (Roopenian and Akilesh, 2007). As a result the free antibody gets recycled to the circulation whereas the antigen enters the degradative pathway (Chaparro-Riggers et al., 2012, Igawa et al, 2010).

The pH-sensitive binding therefore enables the antibody to interact with another antigen and allows the neutralization of multiple antigen molecules per antibody molecule. PH-sensitivity can also increase the half-life of antibodies that address membrane associated targets and internalize and degrade upon e.g. receptor binding. Herein pH-sensitivity can lead to increased half-life, when the antibody gets released during the endosomal acidification.

Several different strategies were published that aim for the engineering of pH-switches in proteins. Histidine (His) scanning by which every single amino acid residue (e.g. within the CDR regions) is mutated to His allows the characterization of single substitution variants and identification of effective mutations. Creation of new variants by combining these substitutions can result in enhanced pH-dependent binding (Murtaugh et al., 2011, Chaparro-Riggers et al., 2012, Igawa et al, 2010). Identification of residues that may contribute to pH-sensitivity upon replacement with histidines by structure-based modeling can help to minimize effort & time that is needed during the histidine scanning approach. Crystal structures are required in order to have a precise idea of residues that are critical for binding and the rational design of pH-switches (Sarkar et al., 2002). Combinatorial histidine scanning library approaches require *in vitro* screening technologies (e.g. phage display or yeast display) to isolate pH-sensitive variants from a large molecule library. Murtaugh and colleagues designed a llama VHH antibody library by using oligonucleotide-directed mutagenesis thereby allowing every residue within the binding interface to sample both histidine residues and wild-type residues of the parental VHH antibody. Towards screening of a M13-phage display library (diversity ~ 10¹²) isolated variants showed KDs between 35-91 nM at pH 7.4 and a ~10⁴fold decrease in binding affinity at pH 5.4 (Murtaugh et al. 2011).

Published European patent application EP 2 275 443 A1 is concerned with methods for improving the pharmacokinetics of antigen-binding molecules and methods for increasing the number of times of antigen-binding of antigen-binding molecules. The document discloses antigen-binding molecules, such as antibodies, with pH-dependent antigen binding, wherein at least one amino acid in said antigen-binding molecule has been mutated to histidine.

The published international patent application WO 2012/065072 A2 discloses liquid aqueous formulations comprising the anti-TNFa antibody adalimumab.

The publication Igawa et al., "Engineered Monoclonal Antibody with Novel Antigen-Sweeping Activity In Vivo", PLoS ONE (2013), vol. 8, no. 5, page e63236 discloses a type of engineered antibody, named "sweeping antibody", which shows both pH-dependent antigen binding and increased binding to cell surface neonatal Fc receptor (FcRn) at neutral pH. The document reports that this type of antibody showed selective elimination of antigen from plasma.

The publication Devanaboyina et al., "The effect of pH dependence of antibody-antigen interactions on subcellular trafficking dynamics", mAbs (2013), vol. 5, no. 6, pages 851-859, describes experiments to study how the pH dependence of antibody-antigen interactions affects intracellular trafficking of antigens. The document mentions the problem that antibodies can prolong the persistence of the targeted antigen through a buffering effect and describes the use of "sweeping antibodies".

Since the recycled free antibody is capable of binding to another antigen, pH-dependent antigen binding would enable a single antibody molecule to repeatedly bind to multiple antigens, in contrast to the conventional approach in which a single antibody can bind to antigen only once.

Therefore, it is a general need to make available antibodies which are more effective with respect to their plasma and serum concentration, which can be achieved by installing pH sensitivity with respect to different cellular action sites of the therapeutic antibody.

### Summary of the Invention

The invention is defined by the appended claims. Any other aspects, configurations or embodiments set forth herein not falling within the scope of the claims are for information only.

The invention provides mutated forms of anti-TNFa antibody adalimumab (Humira^{®}), wherein the mutation consists of substitutions of amino acid residues of the original, non-mutated antibody adalimumab by histidine residues in the complementary determining regions (CDRs) of the heavy and / or light chain variable domains of the antibody. The introduction of histidine residues at defined position in some of the CDRs of adalimumab renders the original antibody much more pH-sensitive or pH-dependent compared to the non-mutated antibody. Interestingly, although the histidine-mutated antibody undergoes a significant loss of binding affinity (up to factor 10 and more of K_{D}) the mutated antibody of the invention is therapeutically significantly more effective compared to its parental non-mutated version, resulting in the possibility to reduce the antibody dosage or the antibody dosing intervals und thus the treatment costs significantly.

It has been shown by the invention that the successful introduction of histidine residues according to the invention by replacement of original amino acid residues in the CDRs of adalimumab or biologically active variants and fragments thereof, does not obey routine rules or mental considerations in view of the desired results regarding degree of pH-sensitivity and binding affinity.

Thus, it was found that histidine-mutated versions of adalimumab are specifically therapeutically effective, if the pH dependent antigen binding results in an antigen dissociation rate (K_{dis}) ratio pH 6 / pH 7 which is 5 - 20 fold higher compared to the respective K_{dis} rate ratio of non-mutated adalimumab, and the binding affinity of the mutated adalimumab is 1 - 25%, preferably 1 - 15%, more preferably 2 - 12% of the binding affinity of the non-histidine mutated adalimumab.

Therefore, the invention provides a human anti-TNFa antibody or an antigen binding fragment thereof with pH dependent antigen binding comprising a variant of the light and heavy chain variable regions of human antibody adalimumab with same or similar biological activity, wherein at least one of the CDR domains of the light and / or the heavy chain variable regions is mutated by replacement of one or more amino acids within said CDR domains by a histidine residue, thus generating a mutated adalimumab or adalimumab variant eliciting a pH dependent antigen binding with an antigen dissociation rate (K_{dis}) ratio pH 6 / pH 7 measured by Bioayer Interferometrie (Octet Red), or other comparable methods, which is at least 5, 10, 15 or 10 fold higher compared to the respective K_{dis} rate ratio of non-mutated adalimumab.

Also disclosed is a respective mutated adalimumab, wherein the mutated antibody or antigen binding fragment thereof has a reduced antigen binding affinity, which is preferably 1 - 15%, respectively 1%, 2%, 3%, 4%, 5%, 6%, 7%, 8%, 9%, 10%, 11%, 12%, 13% 14% or 15% of the binding affinity of non-mutated adalimumab.

The invention provides human anti-TNFa antibodies deriving from adalimumab, wherein original amino acid residues within the CDRs of parental adalimumab were replaced by histidine residues. Interestingly, the most effective histidine-mutated versions of adalimumab comprise histidine mutations within the variable light chain, especially in the CDR1 and CDR3 domains of the light chain. Preferred histidine-mutated adalimumab versions include further or independently a histidine-mutated CDR3 heavy chain.

Disclosed herein is mutated adalimumab or antigen binding fragment thereof comprising a CDR3 heavy chain sequence selected from the group consisting of:
VSY**H**STASSLDY (SEQ ID NO: 12)
VSYLSTA**HH**LDY (SEQ ID NO: 13)
VSY**H**STA**HH**LDY (SEQ ID NO: 14)
V**H**Y**H**STASSLDY (SEQ ID NO: 31), and
V**X₁**Y**X₂**STA**X₃X₄**LDY (SEQ ID NO: 40), wherein X₁ is S or H; X₂ is L or H, X₃ = S or H, and X₄ is S or H, and wherein at least X₁ or X₂ or X₃ or X₄ is H.

Further disclosed herein is mutated adalimumab or antigen binding fragment thereof comprising a CDR3 light chain sequence selected from the group consisting of:
**HH**Y**H**RAPYT (SEQ ID NO: 17)
Q**H**Y**H**RAPY**H** (SEQ ID NO: 18
QR**H**NRAPYT (SEQ ID NO: 38)
**X₁H**Y**H**RAPY**X₂** (SEQ ID NO: 19), wherein X₁ is Q or H, and X₂ is T or H, **X₁X₂X₃X₄**RAPY**X₅** (SEQ ID NO: 41), wherein X₁ is Q or H, X2 is R or H, X3 is Y or H, X4 is N or H, and X₅ is T or H, wherein at least X₁ or X₂ or X₃ or X₄ orX₅ is H.

Further disclosed herein is mutated adalimumab or antigen binding fragment thereof comprising a CDR1 light chain sequence selected from the group consisting of:
RASQGIRN**H**LA (SEQ ID NO: 15),
RASQGIRN**HH**A (SEQ ID NO: 16,
RASQGIRN**X₁X₂**A (SEQ ID NO: 42), wherein X, is Y or H, X₂ is L or H, wherein at least X₁ or X₂ is H.

Further disclosed herein is mutated adalimumab or antigen binding fragment thereof comprising a CDR2 light chain sequence selected from the group consisting of:
AA**H**TLQS (SEQ ID NO: 32)

Also disclosed herein is an embodiment in which the histidine-mutated adalimumab or antigen binding fragment thereof comprises a CDR3 heavy chain sequence as specified above and in the claims, and a CDR1 light chain sequence as specified above and in the claims.

Also disclosed herein is an embodiment in which the histidine-mutated adalimumab or antigen binding fragment thereof comprises a CDR3 heavy chain sequence, and a CDR3 light chain sequence as specified above and in the claims.

Also disclosed herein is an embodiment in which the histidine-mutated adalimumab or antigen binding fragment thereof comprises a CDR3 heavy chain sequence, a CDR2 light chain sequence, and a CDR3 light chain sequence as specified above and in the claims.

Also disclosed herein is an embodiment in which the histidine-mutated adalimumab or antigen binding fragment thereof comprises a CDR3 heavy chain sequence, a CDR1 light chain sequence, and a CDR3 light chain sequence as specified above and in the claims.

Also disclosed herein are versions of histidine-mutated adalimumab which comprise one of the following light chain variable regions:
(i)
(ii)
(iii)
(iv)
(v)
(vi)
(vii)
(viii)
(ix) DIQMTQSPSS LSASVGDRVT ITCRASQGIR N**HX₁**AWYQQKP GKAPKLLIYA ASTLQSGVPS RFSGSGSGTD FTLTISSLQP EDVATYYCQR YNRAPYTFGQ GTKVEIK (SEQ ID NO: 20), wherein X₁ is L or H,
(x) DIQMTQSPSS LSASVGDRVT ITCRASQGIR NYLAWYQQKP GKAPKLLIYA ASTLQSGVPS RFSGSGSGTD FTLTISSLQP EDVATYYC**X₁H** Y**H**RAPY**X₂**FGQ GTKVEIK (SEQ ID NO: 21), wherein X₁ is Q or H and X₂ is T or H
(xi) DIQMTQSPSS LSASVGDRVT ITCRASQGIR NYLAWYQQKP GKAPKLLIYA ASTLQSGVPS RFSGSGSGTD FTLTISSLQP EDVATYYC**HH** Y**H**RAPY**X₁**FGQ GTKVEIK (SEQ ID NO: 22), wherein X₁ is T or H;
(xii) DIQMTQSPSS LSASVGDRVT ITCRASQGIR NYLAWYQQKP GKAPKLLIYA ASTLQSGVPS RFSGSGSGTD FTLTISSLQP EDVATYYC**X₁H** Y**H**RAPY**H**FGQ GTKVEIK (SEQ ID NO: 23), wherein X₁ is Q or H;
(xiii) DIQMTQSPSS LSASVGDRVT ITCRASQGIR N**HX₁**AWYQQKP GKAPKLLIYA ASTLQSGVPS RFSGSGSGTD FTLTISSLQP EDVATYYC**X₂H** Y**H**RAPY**X₃**FGQ GTKVEIK (SEQ ID NO: 24), wherein X1 is L or H, and X₂ is Qor H and Xₐ is T or H;
(xiv) DIQMTQSPSS LSASVGDRVT ITCRASQGIR N**X₁X₂**AWYQQKP GKAPKLLIYA A**X₃**TLQSGVPS RFSGSGSGTD FTLTISSLQP EDVATYYC**X₄X₅ X₆X₇**RAPY**X₈**FGQ GTKVEIK (SEQ ID NO: 43), wherein X₁ is Y or H, X₂ is L or H, X₃is S or H, X₄ is Q or H, Xₛ is R or H, X₆ is Y or H, X₇ is N or H, X₈ is T or H, wherein at least X₁ or X₂ or X₃ or X₄ or X₅ or X₆ or X_{7 or} X₈ is H.

Also disclosed herein are versions of histidine-mutated adalimumab which comprise one of the following heavy chain variable regions:
(i)
(ii)
(iii)
(iv) wherein X₁ is S or H, X₂ is L or H, X₃ is S or H, X₄ is S or H, wherein at least X₁ or X₂ or X₃ or X₄ is H.

Further disclosed herein is histidine-mutated adalimumab which comprises any of the variable light chains as specified above and any one of the variable heavy chain domains as specified above.

A first embodiment of the invention is a respectively histidine-mutated adalimumab comprising the variable heavy chain sequence :
EVQLVESGGG LVQPGRSLRL SCAASGFTFD DYAMHWVRQA PGKGLEWVSA ITWNSGHIDY ADSVEGRFTI SRDNAKNSLY LQMNSLRAED TAVYYCAKVS Y**H**STASSLDY WGQGTLVTVS S (SEQ ID NO: 25) and
the variable light chain sequence:

A second embodiment of the invention is a respectively histidine-mutated adalimumab comprising the variable heavy chain sequence :
EVQLVESGGG LVQPGRSLRL SCAASGFTFD DYAMHWVRQA PGKGLEWVSA ITWNSGHIDY ADSVEGRFTI SRDNAKNSLY LQMNSLRAED TAVYYCAKVS YLSTA**HH**LDY WGQGTLVTVS S (SEQ ID NO: 26) and
the variable light chain sequence:

A third embodiment of the invention is a respectively histidine-mutated adalimumab comprising the variable heavy chain sequence :
EVQLVESGGG LVQPGRSLRL SCAASGFTFD DYAMHWVRQA PGKGLEWVSA ITWNSGHIDY ADSVEGRFTI SRDNAKNSLY LQMNSLRAED TAVYYCAKVS YLSTA**HH**LDY WGQGTLVTVS S (SEQ ID NO: 26) and
the variable light chain sequence:

The histidine-mutated adalimumab antibodies or variants or fragments thereof can further comprise human heavy and/or light constant regions.

In one embodiment they comprise a human IgG, preferably a human IgG1 (such as specified by SEQ ID NO: 11), or IgG2 heavy chain constant region.

In another embodiment of the invention they comprise human kappa light chain constant region.

In another embodiment of the invention, the histidine-mutated adalimumab versions of the invention comprise a human heavy chain constant region, preferably IgG1, wherein the Fc portion is mutated at one or more amino acid positions by replacement of the original amino acid residues by other natural amino acid residues which mediate (increase or decrease) binding of the antibody to FcRn.

The histidine-mutated adalimumab antibodies of the invention can be conjugated to other molecules by recombinant fusion with other polypeptides or proteins such as cytokine, or by chemical linkage to chemical, preferably cytotoxic entities, preferably via linker molecules to form antibody-drug-conjugates (ADCs). Techniques and methods to produce such antibody fusion proteins or antibody drug conjugates are well established in the art.

The invention also provides pharmaceutical compositions suitable for the treatment of inflammatory, autoimmune or cancer diseases comprising a variant of a histidine-mutated anti-TNFa antibody adalimumab or an antigen binding fragment thereof according to the invention, or a respective antibody-drug conjugate or an antibody cytokine fusion protein together with a pharmaceutically acceptable carrier, diluent or excipient.

The invention finally provides the therapeutic use of such histidine-mutated adalimumab or biologically effective and active variant or fragment thereof, or ADCs or fusion proteins thereof, for the manufacture of a medicament for the treatment of TNFa induced inflammatory, autoimmune or cancer diseases, as specified in detail below.

Any references in the description to methods of treatment refer to the compounds, pharmaceutical compositions and medicaments of the present invention for use in a method for treatment of the human (or animal) body by therapy (or for diagnosis).

The histidine-mutated adalimumab versions of the inventions exhibit the following advantageous properties and functions:
➢ Adalimumab binding sites are no longer blocked over a long period of time upon TNFα-binding.
➢ Adalimumab shows significantly improved pH-sensitive binding to the target antigen.
➢ The sTNFa can be released much more efficiently by histidine-mutated adalimumab in the acidified endosome during recycling via FcRn.
➢ Upon binding to the membrane-bound target (mTNFa) initiation of receptor mediated internalization and lysosomal degradation of the mTNFa-adalimumab complex which would lead to elimination of the antibody, can be avoided or reduced.
➢ Improved pharmacokinetics of the TNFa-binding antibody that exhibit pH-dependent target binding
➢ pH-sensitive antibody variants are derived from the parental adalimumab sequence by altering the aminoacid sequences within the variable regions of the antibody.
➢ Incorporation of pH-sensitivity into adalimumab is established by substitutions of aminoacids of the heavy and light chain variable regions with histidines
➢ The pH-sensitivity of adalimumab variants corresponds to increased ratios between kinetic parameters (KD, kdis) at pH 6 / pH 7.4 in comparison to the ratios of parental adalimumab.
➢ In this regard the adalimumab antibodies according to the invention are enabled to neutralize the antigen multiple times
➢ Therefore, the therapeutic effect may be prolonged and may allow less frequent administrations of the antibody and/or administrations at lower doses.

### Details of the Invention

### Tumor necrosis factor α (TNFα):

TNFα is a cytokine that plays a key role in immune responses by initiating defense responses to e.g. some bacterial infections. It acts in a complex network in normal inflammation and is also involved in lymhphoid tissue development (Tracey et al., 2008).

Many different cells produce TNFα including macrophages, T cells, mast cells and granulocytes, to name a few. TNFα is a general term that includes soluble (sTNFa) and transmembrane TNFα (mTNFa). Soluble homotrimers are released by cells after proteolytic cleavage of transmembrane TNFα (homotrimers of 26 kDa monomers) by the metalloprotease TNF-alpha-converting enzyme (TACE) (Wajant et al., 2003). Both forms, the sTNFa or mTNFa interact with two distinct receptors, TNF receptor 1 (TNFR1) and TNF receptor 2 (TNFR2). Both receptors differ in their cellular expression profiles and signaling mechanisms. Complexity of the interaction network increases as mTNFa itself can act either as ligand or receptor to both TNF receptors (Wajant et al., 2003).

During disease, high expression of TNFα triggers and mediates downstream mechanisms that can lead to chronic inflammation and pathogenesis within affected compartments (Tracey et al., 2008). Common consequences of TNFα activities in autoimmune diseases are modulation of immune cell recruitment, cell proliferation, cell death and immune regulation. Other effects like matrix degradation and osteoclastogenesis are more disease specific and may be related to different cell types in the affected tissues (Reviewed in Tracey et al., 2008, Choy & Panayi, 2001; Feldmann, 2002; Schottelius et al., 2004).

TNFα has a particularly important role in the regulation of pathogenic events in e.g. rheumatoid arthritis, crohn's disease and plaque psoriasis and rapidly induces other cytokines (e.g. IL-1β and IL-6) (Tracey et al., 2008). In several inflammatory autoimmune diseases the positive clinical outcome upon TNFa-blockade by therapeutic anti-TNFa antibodies has corroborated the link between excessive TNFα exposition and disease pathologies (Aggawal, 2003; Tracey et al., 2008).

### Adalimumab:

Adalimumab (also known by its trademark name HUMIRA^{®}) is a fully human 148 kDa IgG1κ monoclonal antibody against TNFα that originally was developed by using phage display (Abbott Laboratories, 2014). Since market launch in 2002, adalimumab generated huge sales that achieved $4.6 billion just on the US market in 2012 (Aggarwal, 2014). Adalimumab binds to TNFα with high sub-nanomolar affinity and thereby blocks the interaction between TNFα and p55 and p75 cell surface receptors TNFR1 or TNFR2 (Abbott Laboratories, 2014).

As adalimumab binds also to mTNFa with high affinity further possible modes of action include apoptosis or cytokine suppression upon reverse signaling mechanisms and CDC or ADCC mediated cell killing (Reviewed in Tracey et al., 2008 and Horiuchi et al, 2010).

The amino acid sequences of Adalimumab (and variants thereof) and its pharmacological and therapeutic properties are disclosed, for example in WO 97/029131.

The sequences which are important in view of the present invention are listed below in detail:
Adalimumab full light chain sequence:
Adalimumab light chain sequence, variable region (VL):
Adalimumab CDR1 sequence of VL
   RASQGIRNYLA (SEQ ID NO: 3)
Adalimumab CDR2 sequence of VL
   AASTLQS (SEQ ID NO: 4)
Adalimumab CDR3 sequence of VL
   QRYNRAPYT (SEQ ID NO: 5)
Adalimumab full heavy chain sequence:
Adalimumab heavy chain sequence, variable region (VH):
Adalimumab CDR1 sequence of VH:
   DYAMH (SEQ ID NO: 8)
Adalimumab CDR2 sequence of VH:
   AITWNSGHIDYADSVEG (SEQ ID NO: 9)
Adalimumab CDR3 sequence of VH:
   VSYLSTASSLDY (SEQ ID NO: 10)
Adalimumab human heavy chain IgG1 constant region:

Adalimumab is approved in many countries for a couple of therapeutic treatments, such as rheumatoid arthritis, psoriatic arthritis, ankylosing spondylitis, and Crohn's disease.

Therefore, the histidine-mutated adalimumab versions according to the invention are also applicable in the treatment of rheumatoid arthritis, psoriatic arthritis, ankylosing spondylitis, and Crohn's disease, but also in the treatment of other diseases which are induced or triggered by TNFa. This may include autoimmune disorders as well as cancer diseases.

### Selection of antibodies:

Selection of suitable anti-TNFa antibody versions of adalimumab and fragments thereof according to the present disclosure may be achieved by well established and known methods and techniques in the art, such as by histidine substitution via page display libraries or from combinatorial histidine substitution libraries by yeast surface display. Details are provided in the Example section.

### Terms, Definitions, Details

The term *"antibody"* or *"immunoglobulin"* is used according to the invention in the broadest sense and specifically covers intact monoclonal antibodies, polyclonal antibodies, multispecific antibodies (e.g. bispecific antibodies) formed from at least two intact antibodies, and antibody fragments, so long as they exhibit the desired biological activity. Depending on the amino acid sequence of their constant regions, intact or whole antibodies can be assigned to different classes. There are five major classes of intact antibodies: IgA, IgD, IgE, IgG, and IgM, and several of these may be further divided into "subclasses" (isotypes), e.g., IgG1, IgG2, IgG3, IgG4, IgA, and IgA2.

Preferred major class for antibodies according to the invention is IgG, in more detail IgG1 and IgG2, most preferably IgG1.

*"Antibody fragments"* according to the invention comprise a portion of an intact antibody, preferably comprising the antigen-binding or variable region thereof. Examples of antibody fragments include Fab, Fab', F(ab')2, Fv and Fc fragments, diabodies, linear antibodies, single-chain antibody molecules; bispecific and multispecific antibodies formed from antibody fragment(s).

A *"whole or complete"* antibody according to the invention is an antibody which comprises an antigen-binding variable region as well as a light chain constant domain (CL) and heavy chain constant domains, CH1, CH2 and CH3.

A "Fc" region of an antibody according to the invention comprises, as a rule, a CH2, CH3 and the hinge region of an IgG1 or IgG2 antibody major class. The hinge region is a group of about 15 amino acid residues which combine the CH1 region with the CH2-CH3 region.

A "Fab" fragment also contains the constant domain of the light chain and the first constant domain (CH1) of the heavy chain and has one antigen-binding site only.

A "*Fab*'" fragments differ from Fab fragments by the addition of a few residues at the carboxy-terminus of the heavy chain CH1 domain including one or more cysteine residues from the antibody hinge region.

A "*F(ab')2*" antibody according to this invention is produced as pairs of Fab' fragments which have hinge cysteines between them.

*"Single-chain Fv"* or *"scFv"* antibody fragments according to the invention comprise the V, and V, domains of antibody, wherein these domains are present in a single polypeptide chain. Preferably, the Fv polypeptide further comprises a polypeptide linker between the VH and VL domains which enables the scFv to form the desired structure for antigen binding.

The "variable domain" of an antibody according to the invention comprises the framework regions (usually FR1 to FR4) as well as the CDR domains (usually CDR1, CDR2 and CDR3) which are designated as "hypervariable regions".

The term *"hypervariable region"* or "CDR" when used herein refers to the amino acid residues of an antibody which are responsible for antigen-binding. The hypervariable region generally comprises amino acid residues from a "complementarity determining region" or "CDR" (e.g. residues 24-34 (L1), 50-56 (L2) and 89-97 (L3) in the light chain variable domain and 31-35 (H1), 50-65 (H2) and 95-102 (H3) in the heavy chain variable domain; and/or those residues from a "hypervariable loop" (e.g. residues 26-32 (L1 ), 50-52 (L2) and 91-96 (L3) in the light chain variable domain and 26-32 (H1), 53-55 (H2) and 96-101 (H3) in the heavy chain variable domain; Chothia and Lesk J. Mol. Biol. 196:901-917 (1987)).

If not otherwise pointed out, the amino acid positions within the antibody molecules according to this invention are numbered according to Kabat.

*"Framework Region"* or *"FR"* residues are those variable domain residues other than the hypervariable region residues as herein defined.

*"Antibody variants"* according to the invention include antibodies that have a modified amino acid sequence compared to the parental antibody but have same or changed binding affinity to the targeted antigen. Antibody variants differ from the parental antibody by replacement or deletion or addition of one or more amino acid residues at specific positions within the variable domains, including the CDR domains, and or the constant regions of the antibody, in order to modify certain properties of the antibody, such as binding affinity and/or receptor functions, like ADCC, FcRn binding and the more. The histidine-mutated antibodies of this invention without further modifications are not designated as "antibody variants" according to this invention. Antibody variants according to the invention exhibit a sequence homology of 80 - 99% compared to the parental antibody, preferably 90%, 91%, 92%, 93%, 94%, 95%, 96%, 97%, 98% and 99%, dependent on the specific location of the amino acid residue to be replaced, deleted or added. Antibody variants of adalimumab are, for example, disclosed in The term *"fusion protein"* refers to a natural or synthetic molecule consisting of one ore more biological molecules as defined above, wherein two or more peptide- or protein-based (glycoproteins included) molecules having different specificity are fused together optionally by chemical or amino acid based linker molecules. The linkage may be achieved by C-N fusion or N-C fusion (in 5' → 3' direction), preferably C-N fusion. A fusion protein according to the invention is said fusion of an antibody or antibody variant of this invention fused to another protein or polypeptide, preferably a cytokine.

The term "antibody-drug conjugate (ADC)" refers according to the invention to an immunoconjugate composed of an antibody, preferably complete antibody, according to the invention, and a preferably chemical cytotoxic agent. The components are chemically attached to each other by specific linkers. The antibody of the invention (preferably within its heavy chain constant region) may be modified at one or more amino acid positions in order to create a suitable linkage to the linker and/or the cytotoxic payload drug. Method and techniques to generate such ADCs are well known in the art.

The term "Fc receptor" means, according to the invention, receptors for the Fc region of immunoglobulins (FcRs) that link humoral responses to cellular activities within the immune system. Based on their function, two general groups of FcR can be distinguished: those expressed predominantly by leucocytes that trigger antibody effector functions and those that primarily mediate transport of immunoglobulins across epithelial or endothelial surfaces. ADCC is triggered through interaction of target-bound antibodies (belonging to IgG or IgA or IgE classes) with certain Fc receptors (FcRs). ADCC involving human IgG1 is highly dependent on the glycosylation profile of its Fc portion and on the polymorphism of Fcγ receptors. The term "FcRn" means the specific neonatal Fc receptor, which binds binds IgG at acidic pH of (<6.5) but not at neutral or higher pH. The receptor is responsible for extending half-life of IgG antibodies in serum.

The term *"cytokine"* is a generic term for proteins released by one cell population which act on another cell as intercellular mediators. Examples of such cytokines are lymphokines, monokines, and traditional polypeptide hormones, such as vascular endothelial growth factor (VEGF); integrin; thrombopoietin (TPO); nerve growth factors such as NGFβ; platelet-growth factor; transforming growth factors (TGFs) such as TGFα and TGFβ; erythropoietin (EPO); interferons such as IFNα, IFNβ, and IFNγ; colony stimulating factors such as M-CSF, GM-CSF and G-CSF; interleukins such as IL-1, IL-1a, IL-2, IL-3, IL-4, IL-5, IL-6, IL-7, IL-8, IL-9, IL-10, IL-11, IL-12; and TNF-α or TNF-β.

The term *"biologically*/*functionally effective"* or *"therapeutically effective (amount)"* refers to a drug / molecule which causes a biological function or a change of a biological function in vivo or in vitro, and which is effective in a specific amount to treat a disease or disorder in a mammal, preferably in a human.

The term *"pharmaceutical treatment'* means a variety of modalities for practicing the invention in terms of the steps. For example, the agents according to the invention can be administered simultaneously, sequentially, or separately. Furthermore, the agents can be separately administered within one or more time intervals between administrations. Therapeutic compositions of the present invention contain a physiologically tolerable carrier together with the relevant agent as described herein, dissolved or dispersed therein as an active ingredient.

As used herein, the term *"pharmaceutically acceptable"* refers to compositions, carriers, diluents and reagents which represent materials that are capable of administration to or upon a mammal without the production of undesirable physiological effects such as nausea, dizziness, gastric upset and the like. The preparation of a pharmacological composition that contains active ingredients dissolved or dispersed therein is well understood in the art and need not be limited based on formulation. Typically, such compositions are prepared as injectable preparation either as liquid solutions or suspensions, however, solid forms suitable for solution, or suspensions, in liquid prior to use can also be prepared. The preparation can also be emulsified. The active ingredient can be mixed with excipients which are pharmaceutically acceptable and compatible with the active ingredient and in amounts suitable for use in the therapeutic methods described herein. Suitable excipients are, for example, water, saline, dextrose, glycerol, ethanol or the like and combinations thereof. In addition, if desired, the composition can contain minor amounts of auxiliary substances such as wetting or emulsifying agents, pH buffering agents and the like which may enhance the effectiveness of the active ingredient. The therapeutic composition of the present invention can include pharmaceutically acceptable salts of the components therein.

The histidine-mutated adalimumab versions according to the invention are suitable for the treatment of the same disorders and diseases as the approved and marketed non-histidine mutated adalimumab (HUMIRA^{®}), which are rheumatoid arthritis, juvenile idiopathic arthritis, psoriatic arthritis, ankylosing spondylitis, Crohn's disease, ulcerative colitis and chronic plaque psoriasis, wherein the drug is preferably administered by subcutaneous injection.

Like the marketed drug, the histidine-mutated adalimumab according to the invention can be used alone or in combination with other drugs which support the therapy, such as methotrexate, DMARDS, glucocorticoids, nonsteroidal anti-inflammatory drugs (NSAIDs), and/or analgesics.

The standard dose regimen of HUMIRA^{®} is usually 40mg every week as single dose. The histidine-mutated adalimumab versions according exhibit, as pointed out earlier, a significantly stronger pH-dependency as HUMIRA^{®} , and thus can be administered in doses which correspond only 10 - 90%, in detail 10%, 20%, 30%, 40%, 50%, 60%, 70%, 80% and 90%, of the recommended dose of HUMIRA^{®}, dependent on the respective K_{dis} values of the used histidine-mutated adalimumab versions.

### Short Description of the Figures:

**Figure 1****:** Proposed differences between a) conventional and b) pH-dependent antibody on soluble antigen binding (Modified from: Igawa et al., 2010).

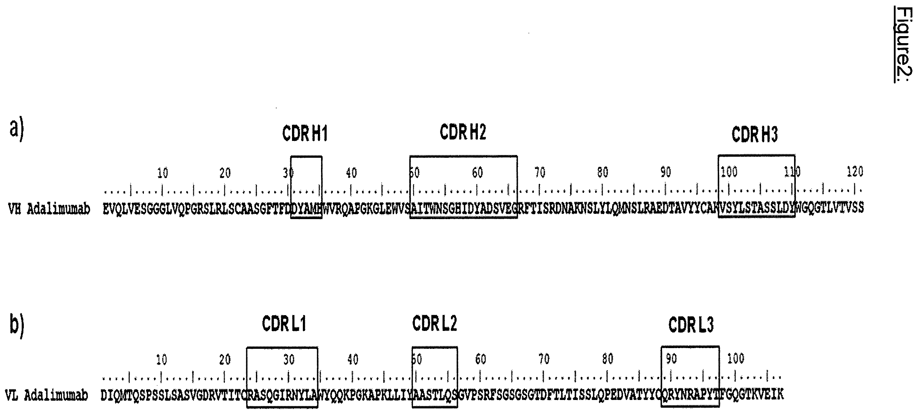

**Figure 2****:** Adalimumab amino acid sequences of the variable regions. The complementary determining regions are highlighted with red boxes. a) Variable region of the heavy chain (VH). b) Variable region of the light chain (VL).

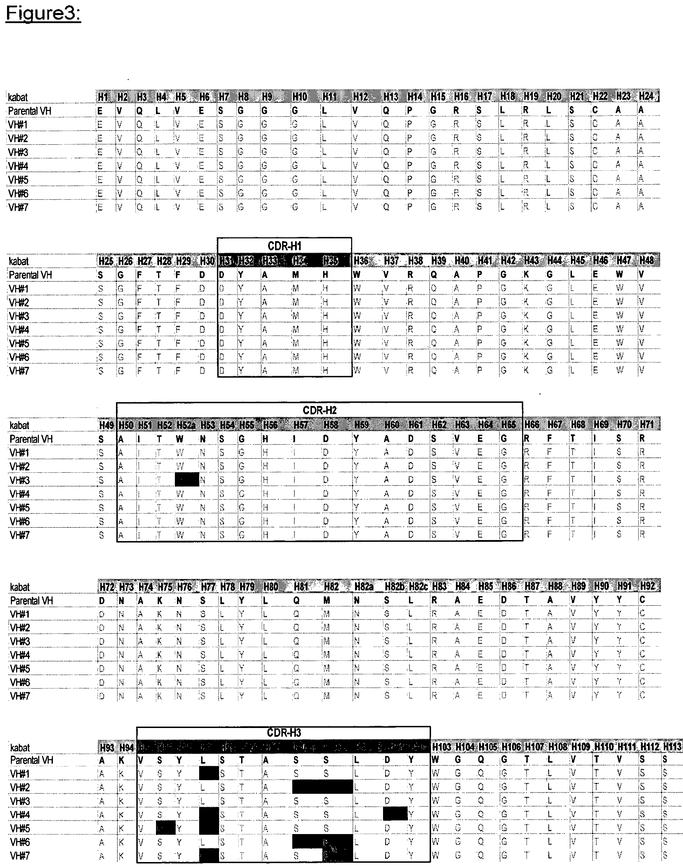

**Figure 3****:** Aligned protein sequences of seven unique VH variants with the parental VH. Unique sequences were isolated from the heavy chain library approach after three rounds of screening. Parental VH sequence is shown on top and residues that vary from the parental VH are highlighted for every variant.

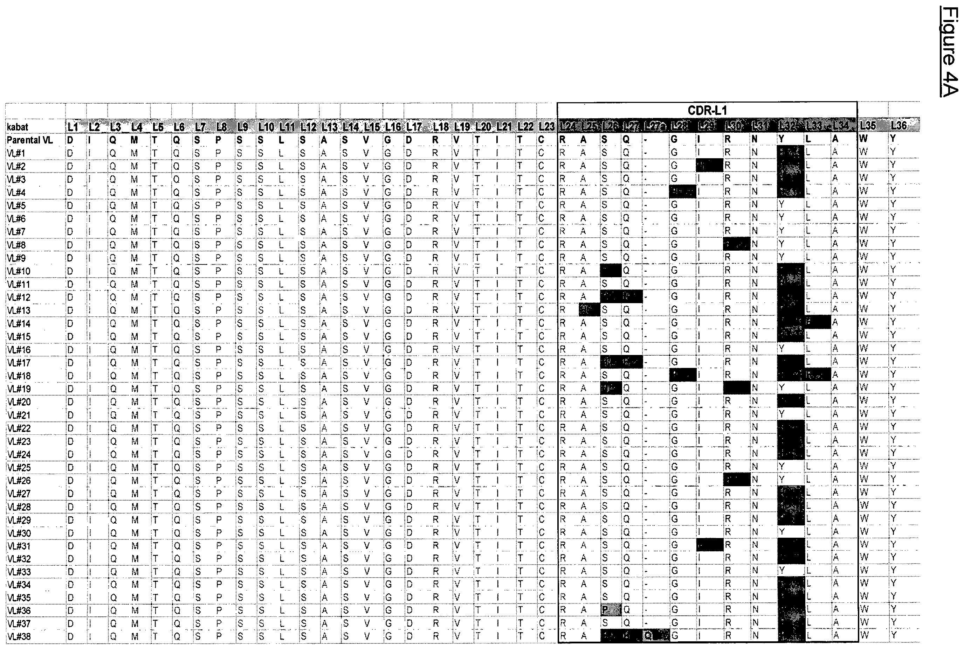

**Figure 4A****:** First part of protein sequence alignment (1-3 parts) of the parental VL and 38 unique VL variants that were isolated from the light chain library after three rounds of screening. Parental VL sequence is shown on top and residues that vary from the parental VL are highlighted for every variant.

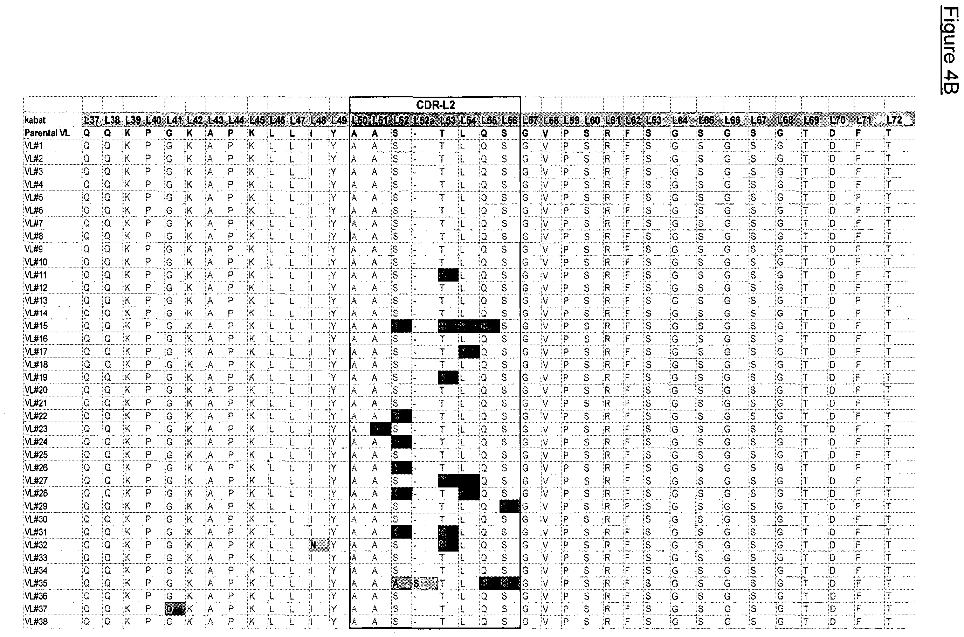

**Figure 4B****:** Second part of protein sequence alignment (1-3 parts) of the parental VL and 38 unique VL variants that were isolated from the light chain library after three rounds of screening. Parental VL sequence is shown on top and residues that vary from the parental VL are highlighted for every variant.

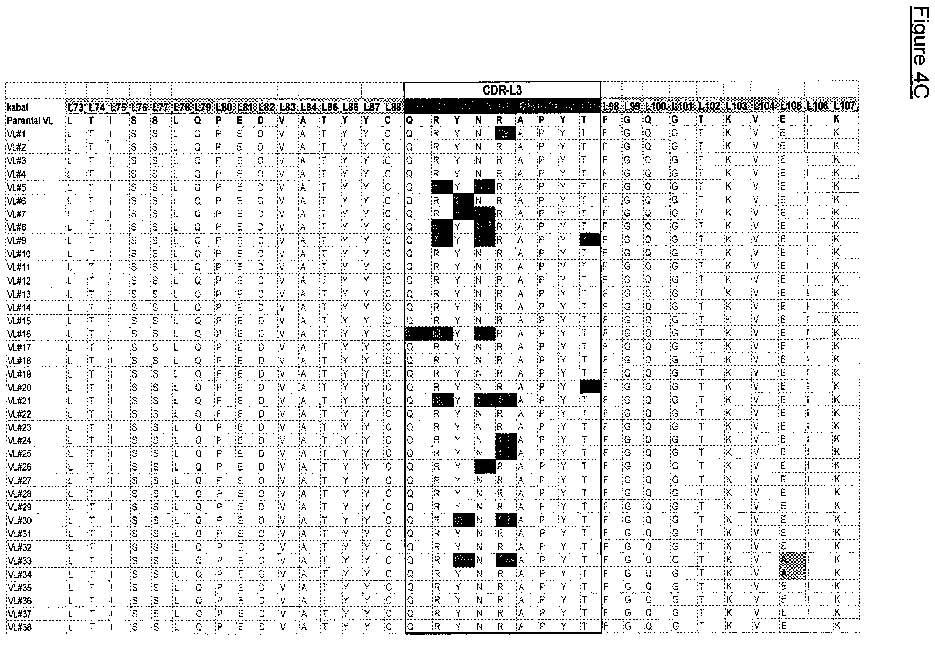

**Figure 4C****:** Third part of protein sequence alignment (1-3 parts) of the parental VL and 38 unique VL variants that were isolated from the light chain library after three rounds of screening. Parental VL sequence is shown on top and residues that vary from the parental VL are highlighted for every variant.

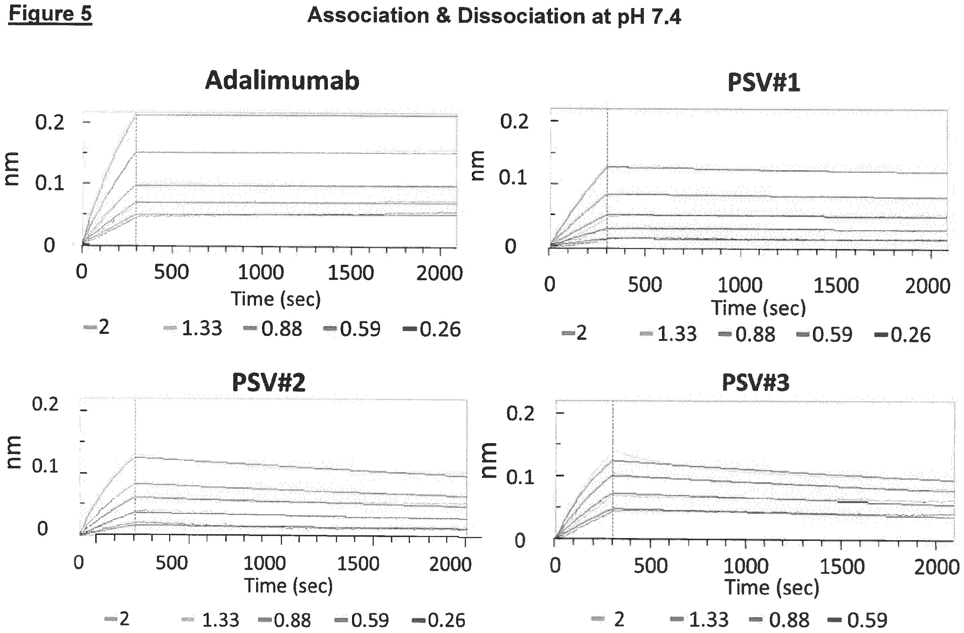

**Figure 5****:** Octet Red sensorgrams of adalimumab and three variants with pH-dependent binding to rhTNFa. Association was done with rhTNFa concentrations ranging between 0.26 nM - 2 nM at pH 7.4 and dissociation was carried out at pH 7.4. Kinetics binding constants were determined through global fitting using Octet 8.0 Software.

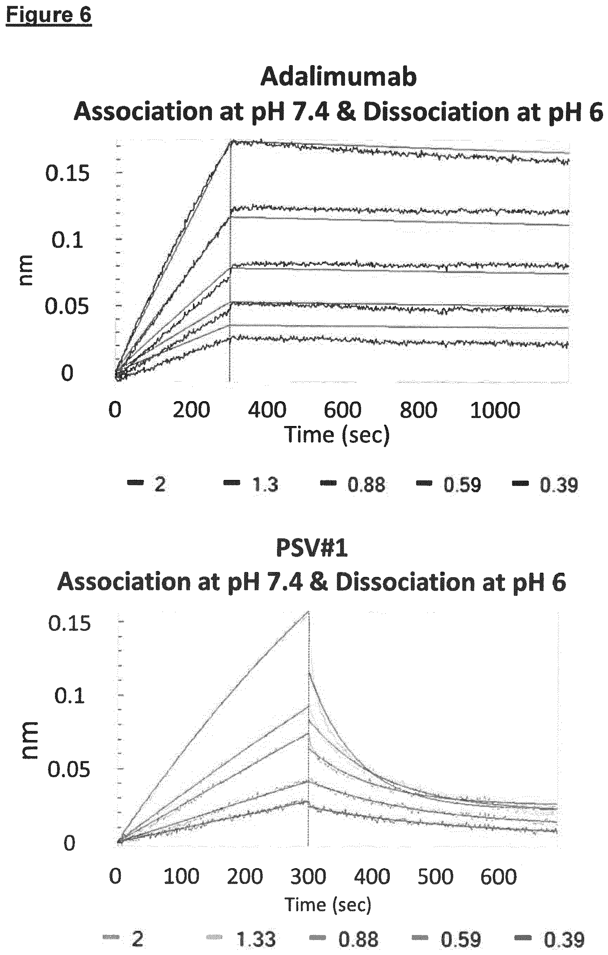

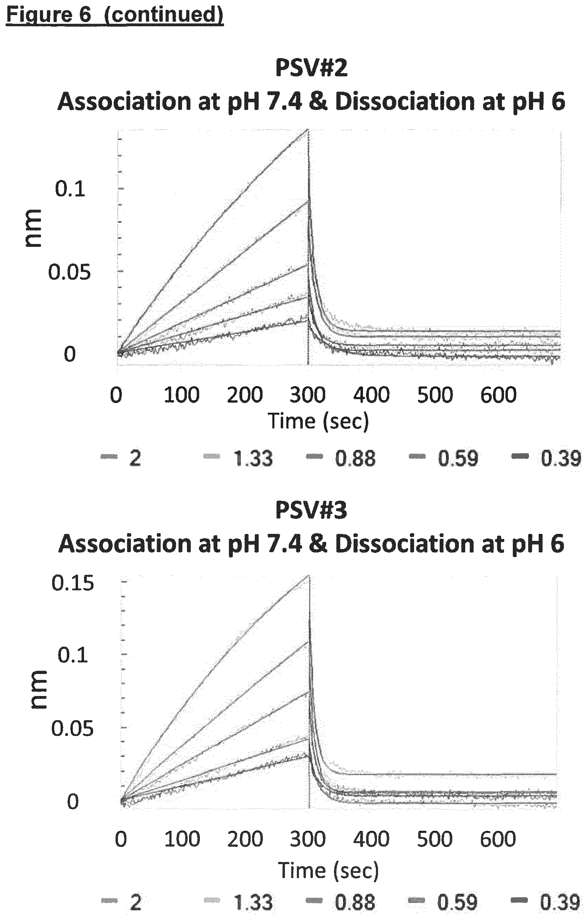

**Figure** 6: Octet Red sensorgrams of adalimumab and three variants with pH-dependent binding. Association was done with rhTNFa concentrations ranging between 0.26 nM - 2 nM at pH 7.4 and dissociation was carried out at pH 6. Off-rates were determined for PSV#1, PSV#2 and PSV#3 through local partial fitting using Octet 8.0 Software. Off-rates for adalimumab were generated by using global fitting.

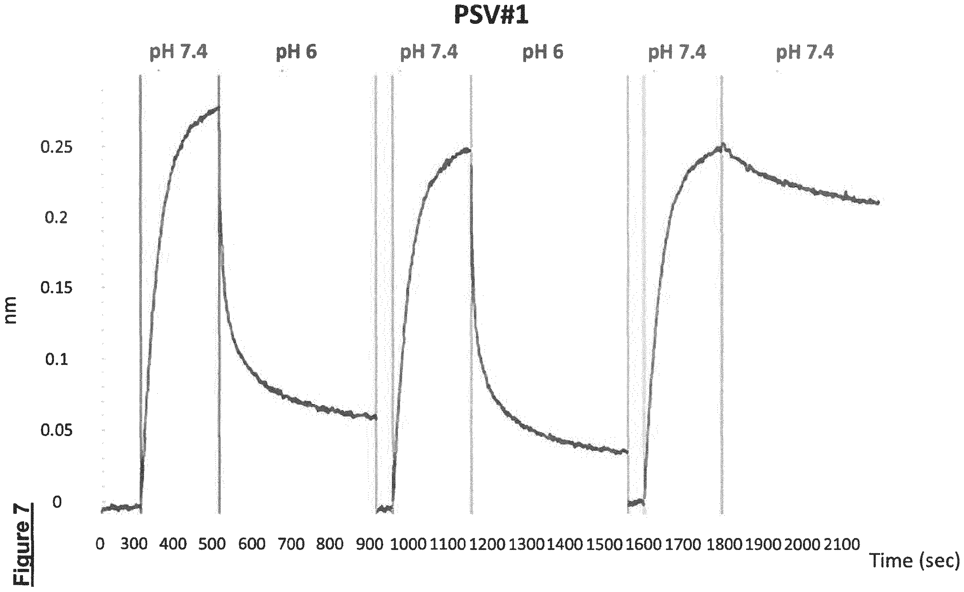

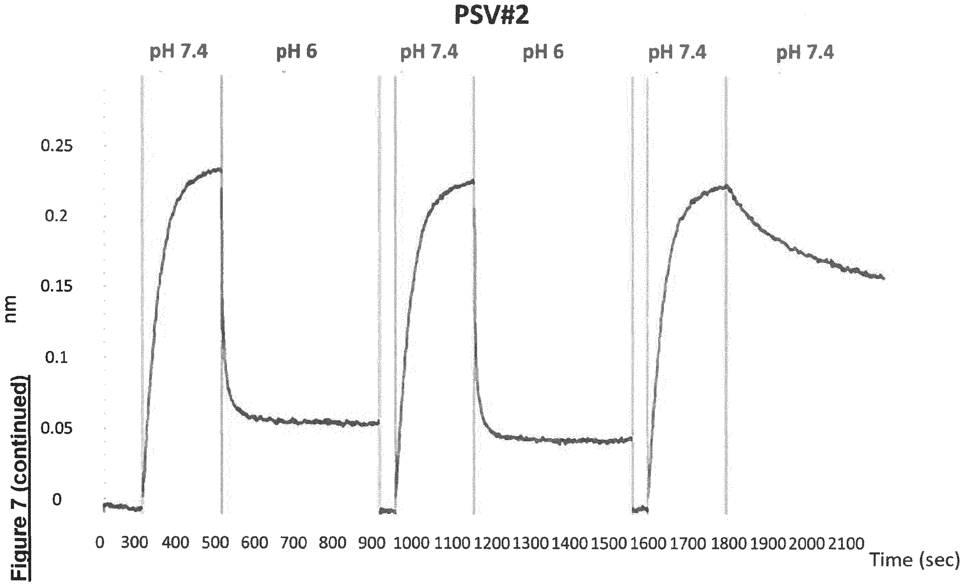

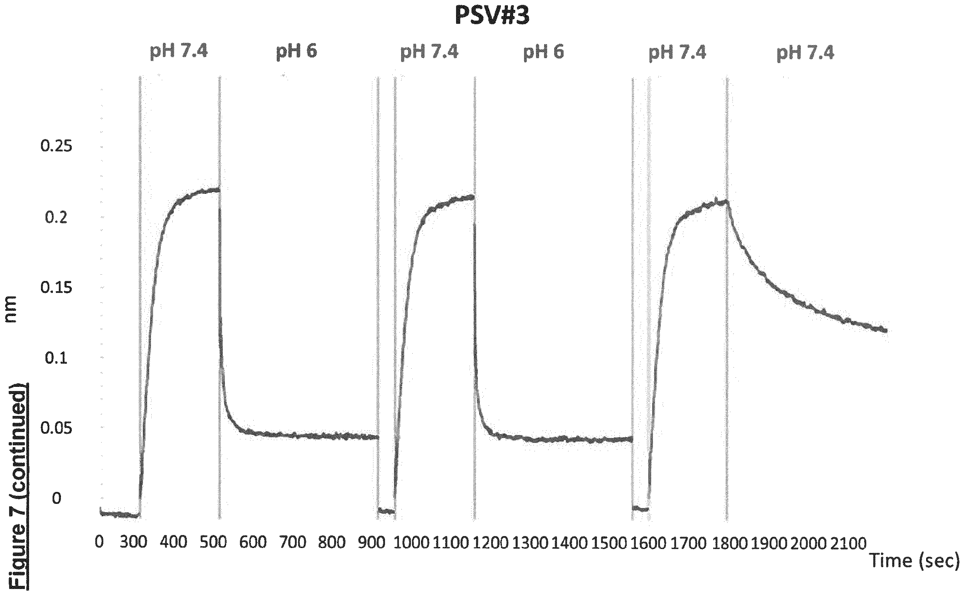

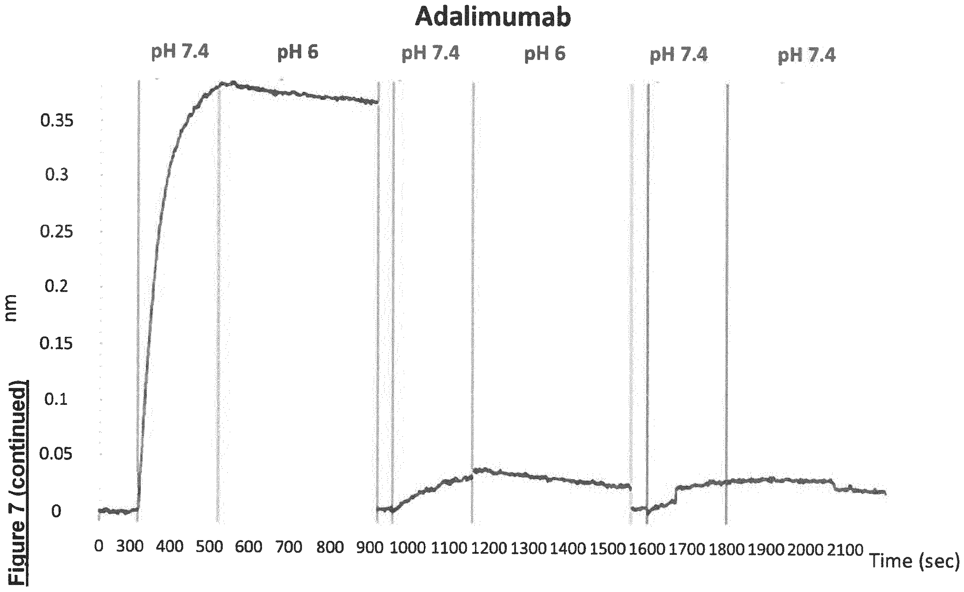

**Figure 7****:** Octet Red sensorgrams of adalimumab and three variants with pH-dependent binding (PSV#1, PSV#2, PSV#3). Adalimumab shows fast association of rhTNFa and maintains tight binding during the dissociation step at pH 6. In contrast, pH-dependent binding variants show reversible rhTNFa binding at pH 7.4 after fast release of rhTNFa during the dissociation step at pH 6. Association to 13 nM rhTNFa was measured for 200 sec and dissociation carried out for 400 sec. After two binding cycles the last dissociation step was done at pH 7.4, showing slow release of rhTNFa.

### REFERENCES

1. Aggarwal B.B., Signalling pathways of the TNF superfamily: a double-edged sword.Nat Rev Immunol. 2003 Sep;3(9):745-56. Review
2. Aggarwal R.S., What's fueling the biotech engine-2012 to 2013.Nat Biotechnol.1 (2014) 32-9.
3. Atmanene C., Wagner-Rousset E., Malissard M., Chol B., Robert A., Corvaïa N., Van Dorsselaer A., Beck A., Sanglier-Cianférani S., Extending mass spectrometry contribution to therapeutic monoclonal antibody lead optimization: characterization of immune complexes using noncovalent ESI-MS. Anal Chem. 15 (2009) 16364-73.
4. Boder E.T., Wittrup K.D., Yeast surface display for screening combinatorial polypeptide libraries, Nat. Biotechnol. 6 (1997) 553-7
5. Carter P.J., Introduction to current and future protein therapeutics: a protein engineering perspective. Exp Cell Res. 9 (2011) 1261-9
6. Chaparro-Riggers J., Liang H., DeVay R.M., Bai L., Sutton J.E., Chen W., Geng T., Lindquist K., Casas M.G., Boustany L.M., Brown C.L., Chabot J., Gomes B., Garzone P., Rossi A., Strop P., Shelton D., Pons J., Rajpal A., Increasing serum half-life and extending cholesterol lowering in vivo by engineering antibody with pH-sensitive binding to PCSK9. J Biol Chem. 14 (2012) 11090-7.
7. Choy, E. H. S., Panayi, G. S., Cytokine pathways and joint inflammation in rheumatoid arthritis. N Engl J Med. 12 (2001) 907-16
8. Dall'Acqua W.F., Kiener P.A., Wu H., Properties of human IgG1s engineered for enhanced binding to the neonatal Fc receptor (FcRn). J Biol Chem 281 (2006) 23514-24.
9. Feldmann, M., Development of anti-TNF therapy for rheumatoid arthritis. Nat Rev Immunol 2 (2002), 364-371
10. Finkelman F.D., Madden K.B., Morris S.C., Holmes J.M., Boiani N., Katona I.M., Maliszewski C.R., Anti-cytokine antibodies as carrier proteins. Prolongation of in vivo effects of exogenous cytokines by injection of cytokine-anti-cytokine antibody complexes.J Immunol. 3 (1993) 1235-44
11. Gera N., Hill A.B., White D.P., Carbonell R.G, Rao B.M., Design of pH sensitive binding proteins from the hyperthermophilic Sso7d scaffold. PLoS One. 7 (2012)
12. Humira (adalimumab) prescribing information. Abbott Laboratories. September 2013. Available at: http://www.rxabbott.com/pdf/humira.pdf.
   Accessed April 21, 2014.
13. Horiuchi T., Mitoma H, Harashima S, Tsukamoto H, Shimoda T. Transmembrane TNF-alpha: structure, function and interaction with anti-TNF agents. Rheumatology (Oxford). 7 (2010) 1215-28
14. Igawa T., Ishii S., Tachibana T., Maeda A., Higuchi Y., Shimaoka S., Moriyama C., Watanabe T., Takubo R., Doi Y., Wakabayashi T., Hayasaka A., Kadono S., Miyazaki T., Haraya K., Sekimori Y., Kojima T., Nabuchi Y., Aso Y., Kawabe Y., Hattori K., Antibody recycling by engineered pH-dependent antigen binding improves the duration of antigen neutralization. Nat Biotechnol.11 (2010) 1203-7
15. Igawa T., Maeda A., Haraya K., Tachibana T., Iwayanagi Y., Mimoto F., Higuchi Y., Ishii S., Tamba S., Hironiwa N., Nagano K., Wakabayashi T., Tsunoda H., Hattori K. ,Engineered monoclonal antibody with novel antigen-sweeping activity in vivo. PLoS One. 8 (2013)
16. Ito W., Sakato N., Fujio H., Yutani K., Arata Y., Kurosawa Y., The His-probe method: effects of histidine residues introduced into the complementarity-determining regions of antibodies on antigen-antibody interactions at different pH values. FEBS Lett.1 (1992) 85-8.
17. Kaymakcalan Z., Sakorafas P., Bose S., Scesney S., Xiong L., Hanzatian D.K., Salfeld J., Sasso E.H., Comparisons of affinities, avidities, and complement activation of adalimumab, infliximab, and etanercept in binding to soluble and membrane tumor necrosis factor. Clin Immunol. 2 (2009) 308-16
18. Kuo T.T., Aveson V.G., Neonatal Fc receptor and IgG-based therapeutics. MAbs. 5 (2011) 422-30
19. Murtaugh M.L., Fanning S.W., Sharma T.M., Terry A.M., Horn J.R., A combinatorial histidine scanning library approach to engineer highly pH-dependent protein switches. Protein Sci. 9 (2011) 1619-31
20. Roopenian D.C., Akilesh S., FcRn: the neonatal Fc receptor comes of age. Nat Rev Immunol. 9 (2007) 715-25
21. Sarkar C.A., Lowenhaupt K., Horan T., Boone T.C., Tidor B., Lauffenburger D.A., Rational cytokine design for increased lifetime and enhanced potency using pH-activated histidine switching. Nat Biotechnol. 9 (2002) 908-13
22. Schabert V.F., Watson C., Joseph G.J., Iversen P., Burudpakdee C., Harrison D.J., Costs of tumor necrosis factor blockers per treated patient using real-world drug data in a managed care population. J Manag Care Pharm. 8 (2013) 621-30.
23. Schottelius, A. J. G., Moldawer, L. L., Dinarello, C. A., Asadullah, K., Sterry, W., & Edwards, C. K. Biology of tumor necrosis factor-alphaimplications for psoriasis. Exp Dermatol 13 (2004) 193-222.
24. Tracey D, Klareskog L, Sasso EH, Salfeld JG, Tak PP. Tumor necrosis factor antagonist mechanisms of action: a comprehensive review. Pharmacol Ther. 2 (2008) 244-79.
25. Wajant H, Pfizenmaier K, Scheurich P. Tumor necrosis factor signaling. Cell Death Differ. 1 (2003) 45-65.

### EXAMPLES

### Example 1: Selection of pH-sensitive anti-TNFα antibodies derived from adalimumab

Selection of anti-TNFa antibody fragments from combinatorial histidine substitution libraries by yeast surface display: Based on the heavy and light chains of adalimumab, two antibody libraries were synthesized by Geneart, Regensburg by using pre-assembled trinucelotides building blocks. During the synthesis either parental or histidine residues were sampled whereby sampling of histidines was restricted to the complementary determining regions (CDRs) of the heavy and light chains. Most adalimumab library members carried three histidine residues that were spread over all three CDRs (Fig. 2) but variants were also synthesized that carried more or less histidine substitutions (ranging between 0 to ~20). Theoretical diversities: Heavy chain library ~10'000 variants, light chain library ~3000 variants.

Both libraries were separately subcloned into plasmid vectors by gap-repair cloning in the EBY100 yeast strain that allows covalent yeast surface display of antibody Fab-fragments (Boder and Wittrup, 1997). Corresponding parental chains were paired with the heavy or light chain libraries and the two resulting libraries were separately screened by fluorescence activated cell sorting (FACS). Cells that carried pH-sensitive adalimumab variants were subsequently enriched over three rounds of screening by applying a specific staining & selection strategy (not explained here).

Fab-fragments were selected that do reversible high affinity (KD within sub nanomolar ranges) binding to recombinant human TNFα (rhTNFa) at pH 7.4, once after rhTNFa has been released within 30 minutes at pH 6. After three rounds of screening for variants that bind to rhTNFa in pH-dependent manner, sequence analysis of isolated single clones revealed variants that carried specific histidine substitutions patterns (shown in Figure 3 & 4). One mutational hot-spot was identified within the CDR3 region of the heavy chain sequence data set. Two mutational hot-spots were identified within CDR-L1 and CDR-L3 regions of the light chain sequence data set. Only abundant heavy and light chain variants (occurrence within analyzed sequence set: N>1) were selected for further processing & characterization, resulting in eight light chain and three heavy chain candidates shown in Table 1 and Table 2:

**Table 1. Three abundant (N> 1) heavy chain sequences within 38 isolated single clones after three rounds of screening.**

| **Abundance** | **Number of His substitutions (Region)** | **Variant code** |
|---|---|---|
| 29/38 | 1 (CDR H3) | VH#1 |
| 3/38 | 2 (CDR H3) | VH#2 |
| 2/38 | 2 (CDR H3) | VH#5 |

**Table 2. Eight abundant (N>1) light chain sequences within 98 isolated single clones after three rounds of screening.**

| **Abundance** | **Number of His substitutions (Region)** | **Variant code** |
|---|---|---|
| 36/98 | 1 (CDR L1) | VL#3 |
| 13/98 | 2 (CDR L3) | VL#5 |
| 5/98 | 3 (CDR L3) | VL#9 |
| 4/98 | 3 (CDR L3) | VL#16 |
| 3/98 | 2 (CDR L1) | VL#14 |
| 3/98 | 1 (CDR L3) | VL#6 |
| 2/98 | 1 (CDR L1) 1 (CDR L3) | VL#1 |
| 2/98 | 1 (CDR L1) 1 (CDR L2) | VL#22 |

Variable regions of abundant VH/VL variants as well as parental adalimumab sequences were cloned into vectors that allow expression of full length IgG1κ molecules in mammalian cells (HEK293 & Expi293). All possible combinations of heavy and light chain variants were co-expressed in mammalian cells. Herein 24 heavy chain and light chain variant combinations were expressed as well as 11 IgG species that derived from combinations of the heavy or light chain variants with the corresponding parental chains. Initial Octet Red experiments with immobilized antibodies assessed differential dissociation behavior at pH 6 or pH 7.4 after associating rhTNFa at pH 7.4. Ten variants were selected according to their binding profiles in regard of high affinity binding at pH 7.4 (association / dissociation at pH 7.4) and fast release of the antigen at pH 6 (association at pH 7.4 / dissociation at pH 6). For further characterization antibodies were purified via protein-A purification from crude supernatants and finally buffer was exchanged to PBS. Further octet assays revealed differences in binding at pH 7.4 and differences in rhTNFa release at pH 6.

Subsequently three variants (IDs according to figures 3-4: VH#2 + VL#9: **PSV#1,** VH#2 + VL#16: **PSV#2** and VH#1+ VL#14: **PSV#3**) were selected for final characterization on Octet Red.

Binding characteristics of several histidine-mutated adalimumab variants were analyzed in Octet Red experiments. Different histidine mutations in heavy and light chains as well as different heavy and light chain variant combinations have been shown to affect binding affinities at pH 7.4 and the dissociation rates at pH 6.

A combination of several mutations including **Leu98His** in the heavy chain and **Tyr32His, Leu33His** in the light chain generated **PSV#3.** Light chain mutations **Gln89His**, **Arg90His** and **Asn92His** generated the light chain of **PSV#2.** The mutations **Arg90His, Asn92His** and **Thr97His** generated the light chain of **PSV#1.** For both, PSV#1 and PSV#2, mutations of **Ser100.bHis** and **Ser100.cHis** generated the heavy chain.

*(The sequences were numbered* as *shown in* *figures 3* *and* *4a**-c according to kabat numbering. For this purpose, sequences of the variants were aligned together with the parental sequence by using Clustal VV and numbering was applied considering the rules for kabat numbering.)*

Representative sensorgrams of the Octet Red measurements are shown in figure 5 & 6 and corresponding mean values (N=3) of calculated kinetic parameters for adalimumab, PSV#1, PSV#2 and PSV#3 are shown in table 4.

Table 3. *Binding kinetics of adalimumab and pH-dependent binding variants to rhTNFα at pH 7.4 and pH 6. Association rate **(kon),** dissociation rate **(kdis)** and binding affinity **(KD)** of adalimumab, PSV#1, PSV#2 and PSV#3 at pH 7.4. Dissociation rates were determined also at pH 6. Experiments were done at* 25°C *and for every experiment mean values of triplicates are shown (exception: Adalimumab was measured at pH 6 in duplicates). Representative sensorgrams that correspond to the data are shown in* *figures 7* *and 8.*

| | pH 7.4 | | | pH 6 | | |
|---|---|---|---|---|---|---|
| Antibody | KD (M) | kon (M⁻¹s⁻¹) | kdis (s⁻¹) | kdis (s⁻¹) | kdis ratio pH 6 / pH 7.4 | kdis ratio (pH 6) vs. adalimumab |
| Adalimumab | 0.46E-11 | 1.32E+06 | 0.55E-05 | 4.81E-05 | 9 | 1 |
| PSV#1 | 4.63E-11 | 0.67E+06 | 3.26E-05 | 754E-05 | 231 | 157 |
| PSV#2 | 7.73E-11 | 1.14E+06 | 9.35E-05 | 7340E-05 | 785 | 1527 |
| PSV#3 | 11.2E-11 | 1.93E+06 | 21.8E-05 | 11000E-05 | 505 | 2293 |

Parental adalimumab binds with high affinity to rhTNFa at pH 7.4 that was also shown in Kaymakcalan et al., 2009. For the three variants the increasing kdis-values result in a decrease in binding affinity (10-24fold decrease in KD), however interaction with TNFα with picomolar binding affinities in the three-diget range still represents very tight binding (figure 5 and table 3).

Octet Red measurements were also performed to assess the antibodies' pH-sensitivities. Improved antibody efficacy in context of the FcRn-mediated recycling requires tight binding to TNFα in the circulation at pH 7.4 and its fast release in the acidic endosome. In order to evaluate the release of TNFα within the acidic endosome, dissociation was measured at pH 6 after association of rhTNFa at pH 7.4 (figure 6 and table 3). Ratios of dissociation rates at pH 6 and pH 7.4 were determined and all variants showed considerably increased dissociation at pH 6 (59-160fold increased kdis at pH 6, in contrast to adalimumab with a kdis ratio (pH6/pH7.4) of 6 (table 3).

One additional experiment addressed the ability of PSV#1, PSV#2 and PSV#3 to reversibly bind rhTNFa at pH 7.4, after dissociation at pH 6. To ensure that incubation at pH 6 does not irreversibly change TNFα binding capabilities, two cycles of binding (pH 7.4) and release (pH 6) were performed (figure 7). As shown in figure 8, PSV#1, PSV#2 and PSV#3 can reversibly bind after TNFα has been released at pH 6. In contrast to that, adalimumab maintains tight binding during incubation at pH 6.

### Example 2: In vivo characterization of mutants

The effect of parental IgG construct and mutants thereof on the PK of human TNFα was investigated in heterozygous transgenic human FcRn mice, line 176, as well as in homozygous line 32 mice. The former line was more suitable to investigate PK differences between administered IgG constructs, while the latter mouse line provided a better FcRn protection, resulting in longer half-lives, closer to what was expected in human. This longer residence of the scavenger allowed better to evaluate the impact of the antibody on the clearance of the TNFα.

Human TNFa and scavenger were administered by SC route in predefined ratios. Plasma concentration profiles of both total scavenger and total hTNFa was investigated. pH-dependent hTNFa binding was expected to result in increased clearance of the cytokine and decreased clearance of the scavenger.

Selected tissues were collected from the mice, in order to investigate distribution of the scavenger and the target cytokine. Parental IgG were used as reference compound in this study.

The *in vitro* and *in vivo* data sets were used to establish correlations between:
- physico-chemical properties and *in vivo* pharmacokinetics
- FcRn affinity and in in vivo pharmacokinetics and tissue distribution

The correlations were used to build a physiologically-based pharmacokinetic (PBPK) model capable of characterizing and simulating plasma and tissue pharmacokinetics.

## Claims

1. A human antibody or an antigen binding fragment thereof with pH dependent antigen binding comprising a variant of the light and heavy chain variable regions of human antibody adalimumab with same or similar biological activity,
said antibody comprising
the variable heavy chain sequence: and
the variable light chain sequence: or
the variable heavy chain sequence: and
the variable light chain sequence: or
the variable heavy chain sequence: and
the variable light chain sequence:

2. The human antibody of claim 1 comprising human heavy chain IgG1 constant region as specified by SEQ ID NO: 11.

3. The human antibody of claim 2, wherein the Fc portion of said IgG1 constant region is mutated at one or more amino acid positions resulting in a respective antibody with modified FcRn binding.

4. The complete human antibody of claim 2 or 3, comprising human kappa constant region.

5. An antibody-drug conjugate comprising an antibody or antibody fragment of any one of the claims 1 - 4 linked directly or indirectly to a cytotoxic chemical drug or recombinantly fused to a cytokine.

6. A pharmaceutical composition suitable for the treatment of inflammatory, autoimmune or cancer diseases comprising a human antibody or an antigen binding fragment thereof of any one of the claims 1 - 4, or an antibody-drug conjugate of claim 5 together with a pharmaceutically acceptable carrier, diluent or excipient.

7. A human antibody, or an antigen binding fragment thereof, of any one of the claims 1 - 4, or an antibody-drug conjugate of claim 5, for use in the treatment of TNFα induced inflammatory, autoimmune or cancer diseases.

## Patentansprüche

1. Menschlicher Antikörper oder ein antigenbindendes Fragment davon mit pHabhängiger Antigenbindung, umfassend eine Variante der variablen Leicht- und Schwerkettenregionen des menschlichen Antikörpers Adalimumab mit gleicher oder ähnlicher biologischer Aktivität, wobei der Antikörper umfasst
die variable Schwerkettensequenz: und
die variable Leichtkettensequenz: oder
die variable Schwerkettensequenz: und
die variable Leichtkettensequenz: oder
die variable Schwerkettensequenz: und
die variable Leichtkettensequenz:

2. Menschlicher Antikörper nach Anspruch 1, der die durch SEQ ID NO: 11 angegebene konstante Schwerkettenregion des menschlichen IgG1 umfasst.

3. Menschlicher Antikörper nach Anspruch 2, wobei der Fc-Teil der konstanten Region von IgG1 an einer oder mehr Aminosäurepositionen mutiert ist, was zu einem entsprechenden Antikörper mit modifizierter FcRn-Bindung führt.

4. Vollständiger menschlicher Antikörper nach Anspruch 2 oder 3, umfassend die menschliche konstante Region Kappa.

5. Antikörper-Wirkstoff-Konjugat, umfassend einen Antikörper oder ein Antikörperfragment nach irgendeinem der Ansprüche 1 - 4, der/das direkt oder indirekt mit einem cytotoxischen chemischen Wirkstoff verknüpft oder rekombinant an ein Zytokin fusioniert ist.

6. Pharmazeutische Zusammensetzung, die zur Behandlung von Entzündungs-, Autoimmun- oder Krebserkrankungen geeignet ist, umfassend einen menschlichen Antikörper oder ein antigenbindendes Fragment davon nach irgendeinem der Ansprüche 1 - 4 oder ein Antikörper-Wirkstoff-Konjugat nach Anspruch 5 zusammen mit einem pharmazeutisch unbedenklichen Träger, Verdünnungsmittel oder Exzipienten.

7. Menschlicher Antikörper oder ein antigenbindendes Fragment davon nach irgendeinem der Ansprüche 1 - 4 oder ein Antikörper-Wirkstoff-Konjugat nach Anspruch 5 zur Verwendung bei der Behandlung von TNFα-induzierten Entzündungs-, Autoimmun- oder Krebserkrankungen.

## Revendications

1. Anticorps humain ou fragment de liaison à l'antigène de celui-ci ayant une liaison à l'antigène dépendante du pH, comprenant un variant des régions variables de chaîne lourde et légère de l'anticorps humain adalimumab avec une activité biologique identique ou similaire, ledit anticorps comprenant
la séquence de chaîne lourde variable : et
la séquence de chaîne légère variable : ou
la séquence de chaîne lourde variable : et
la séquence de chaîne légère variable : ou
la séquence de chaîne lourde variable : et
la séquence de chaîne légère variable :

2. Anticorps humain selon la revendication 1, comprenant une région constante d'IgG1 de chaîne lourde humaine telle que spécifiée par SEQ ID NO: 11.

3. Anticorps humain selon la revendication 2, dans lequel la portion Fc de ladite région constante d'IgG1 est mutée au niveau d'une ou plusieurs positions d'acides aminés, conduisant à un anticorps respectif ayant une liaison à FcRn modifiée.

4. Anticorps humain complet selon la revendication 2 ou 3, comprenant une région constante kappa humaine.

5. Conjugué anticorps-médicament comprenant un anticorps ou un fragment d'anticorps selon l'une quelconque des revendications 1 - 4, lié directement ou indirectement à un médicament chimique cytotoxique ou fusionné de manière recombinée à une cytokine.

6. Composition pharmaceutique convenable pour le traitement de maladies inflammatoires, auto-immunes ou cancéreuses, comprenant un anticorps humain ou un fragment de liaison à l'antigène de celui-ci selon l'une quelconque des revendications 1 - 4, ou un conjugué anticorps-médicament selon la revendication 5, conjointement avec un véhicule, un diluant ou un excipient pharmaceutiquement acceptable.

7. Anticorps humain ou fragment de liaison à l'antigène de celui-ci selon l'une quelconque des revendications 1 - 4, ou conjugué anticorps-médicament selon la revendication 5, pour une utilisation destinée au traitement de maladies inflammatoires, auto-immunes ou cancéreuses induites par le TNFa.


### SEQUENCE LISTING

<110> Merck Patent GmbH
<120> Anti-TNFa antibodies with pH-dependent Antigen Binding
<130> P14-123
<160> 44
<170> PatentIn version 3.5
<210> 1
   <211> 214
   <212> PRT
   <213> Artificial sequence
<220>
   <223> Adalimumab full light chain sequence
<400> 1
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asn Arg Ala Pro Tyr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 2
   <211> 107
   <212> PRT
   <213> Artificial Sequence
<220>
   <223> Adalimumab light chain sequence, variable region (VL)
<400> 2
<210> 3
   <211> 11
   <212> PRT
   <213> Artificial Sequence
<220>
   <223> Adalimumab CDR1 sequence of VL
<400> 3
   Arg Ala Ser Gln Gly Ile Arg Asn Tyr Leu Ala
   1               5                   10
<210> 4
   <211> 7
   <212> PRT
   <213> Artificial sequence
<220>
   <223> Adalimumab CDR2 sequence of VL
<400> 4
   Ala Ala Ser Thr Leu Gln Ser
   1 5
<210> 5
   <211> 9
   <212> PRT
   <213> Artificial Sequence
<220>
   <223> Adalimumab CDR3 sequence of VL
<400> 5
   Gln Arg Tyr Asn Arg Ala Pro Tyr Thr
   1 5
<210> 6
   <211> 451
   <212> PRT
   <213> Artificial sequence
<220>
   <223> Adalimumab full heavy chain sequence
<400> 6
   Glu Val Gln Leu Val Glu Ser Gly Gly Gly Leu Val Gln Pro Gly Arg
   1 5 10 15
   Ser Leu Arg Leu Ser Cys Ala Ala Ser Gly Phe Thr Phe Asp Asp Tyr
   20 25 30
   Ala Met His Trp Val Arg Gln Ala Pro Gly Lys Gly Leu Glu Trp Val
   35 40 45
   Ser Ala Ile Thr Trp Asn Ser Gly His Ile Asp Tyr Ala Asp Ser Val
   50 55 60
   Glu Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Lys Val Ser Tyr Leu Ser Thr Ala Ser Ser Leu Asp Tyr Trp Gly
   100 105 110
   Gln Gly Thr Leu Val Thr Val Ser Ser Ala Ser Thr Lys Gly Pro Ser
   115 120 125
   Val Phe Pro Leu Ala Pro Ser Ser Lys Ser Thr Ser Gly Gly Thr Ala
   130 135 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Asp Glu Leu Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
   420 425 430
   His Glu Ala Leu His Asn His Tyr Thr Gln Lys Ser Leu Ser Leu Ser
   435 440 445
   Pro Gly Lys
   450
<210> 7
   <211> 121
   <212> PRT
   <213> Artificial Sequence
<220>
   <223> Adalimumab heavy chain sequence, variable region (VH)
<400> 7
<210> 8
   <211> 5
   <212> PRT
   <213> Artificial sequence
<220>
   <223> Adalimumab CDR1 sequence of VH
<400> 8
   Asp Tyr Ala Met His
   1               5
<210> 9
   <211> 17
   <212> PRT
   <213> Artificial sequence
<220>
   <223> Adalimumab CDR2 sequence of VH
<400> 9
   Ala Ile Thr Trp Asn Ser Gly His Ile Asp Tyr Ala Asp Ser Val Glu
   1               5                   10                  15
   Gly
<210> 10
   <211> 12
   <212> PRT
   <213> Artificial sequence
<220>
   <223> Adalimumab CDR3 sequence of VH
<400> 10
   Val Ser Tyr Leu Ser Thr Ala Ser Ser Leu Asp Tyr
   1               5                   10
<210> 11
   <211> 330
   <212> PRT
   <213> Artificial Sequence
<220>
   <223> Adalimumab human heavy chain IgG1 constant region
<400> 11
<210> 12
   <211> 12
   <212> PRT
   <213> Artificial Sequence
<220>
   <223> adalimumab mutated CDR3 heavy chain
<400> 12
   Val Ser Tyr His Ser Thr Ala Ser Ser Leu Asp Tyr
   1               5                   10
<210> 13
   <211> 12
   <212> PRT
   <213> Artificial Sequence
<220>
   <223> Adalimumab mutated CDR3 heavy chain
<400> 13
   Val Ser Tyr Leu Ser Thr Ala His His Leu Asp Tyr
   1 5 10
<210> 14
   <211> 12
   <212> PRT
   <213> Artificial Sequence
<220>
   <223> Adalimumab mutated CDR3 heavy chain
<400> 14
   Val Ser Tyr His Ser Thr Ala His His Leu Asp Tyr
   1 5 10
<210> 15
   <211> 11
   <212> PRT
   <213> Artificial sequence
<220>
   <223> Adalimumab mutated CDR1 light chain
<400> 15
   Arg Ala Ser Gln Gly Ile Arg Asn His Leu Ala
   1 5 10
<210> 16
   <211> 11
   <212> PRT
   <213> Artificial Sequence
<220>
   <223> Adalimumab mutated CDR1 light chain
<400> 16
   Arg Ala Ser Gln Gly Ile Arg Asn His His Ala
   1 5 10
<210> 17
   <211> 9
   <212> PRT
   <213> Artificial Sequence
<220>
   <223> Adalimumab mutated CDR3 light chain
<400> 17
   His His Tyr His Arg Ala Pro Tyr Thr
   1               5
<210> 18
   <211> 9
   <212> PRT
   <213> Artificial Sequence
<220>
   <223> Adalimumab mutated CDR3 light chain
<400> 18
   Gln His Tyr His Arg Ala Pro Tyr His
   1               5
<210> 19
   <211> 9
   <212> PRT
   <213> Artificial Sequence
<220>
   <223> Adalimumab mutated CDR3 light chain, wherein X1 is Q or H and x2 is T or H
<220>
   <221> X1
   <222> (1)..(1)
   <223> X1 is Q or H
<220>
   <221> X2
   <222> (9)..(9)
   <223> X2 is T or H
<400> 19
   Xaa His Tyr His Arg Ala Pro Tyr Xaa
   1               5
<210> 20
   <211> 107
   <212> PRT
   <213> Artificial sequence
<220>
   <223> histidine-mutated adalimumab light chain variable region, wherein X1 is L or H
<220>
   <221> X1
   <222> (33)..(33)
   <223> X1 is L or H
<400> 20
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn His
               20                  25                  30
   Xaa Ala Trp Tyr Gln Gln Lys Pro Gly Lys Ala Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asn Arg Ala Pro Tyr
                   85                  90                  95
   Thr Phe Gly Gln Gly Thr Lys Val Glu Ile Lys
               100                 105
<210> 21
   <211> 107
   <212> PRT
   <213> Artificial Sequence
<220>
   <223> histidine-mutated adalimumab light chain variable region, wherein X1 is Q or H and X2 is T or H
<220>
   <221> X1
   <222> (89)..(89)
   <223> X1 is Q or H
<220>
   <221> X2
   <222> (97)..(97)
   <223> x2 is T or H
<400> 21
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Xaa His Tyr His Arg Ala Pro Tyr
                   85                  90                  95
   Xaa Phe Gly Gln Gly Thr Lys Val Glu Ile Lys
               100                 105
<210> 22
   <211> 107
   <212> PRT
   <213> Artificial Sequence
<220>
   <223> histidine-mutated adalimumab light chain variable region, wherein X1 is T or H
<220>
   <221> X1
   <222> (97)..(97)
   <223> X1 is T or H
<400> 22
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Arg Asn Tyr
   20 25 30
   Leu Ala Trp Tyr Gln Gln Lys Pro Gly Lys Ala Pro Lys Leu Leu Ile
   35 40 45
   Tyr Ala Ala Ser Thr Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Val Ala Thr Tyr Tyr Cys His His Tyr His Arg Ala Pro Tyr
   85 90 95
   Xaa Phe Gly Gln Gly Thr Lys Val Glu Ile Lys
   100 105
<210> 23
   <211> 107
   <212> PRT
   <213> Artificial sequence
<220>
   <223> histidine-mutated adalimumab light chain variable region, wherein X1 is Q or H
<220>
   <221> X1
   <222> (89)..(89)
   <223> X1 is Q or H
<400> 23
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Xaa His Tyr His Arg Ala Pro Tyr
                   85                  90                  95
   His Phe Gly Gln Gly Thr Lys Val Glu Ile Lys
               100                 105
<210> 24
   <211> 107
   <212> PRT
   <213> Artificial sequence
<220>
   <223> histidine-mutated adalimumab light chain variable region, ), wherein X1 is L or H, and X2 is Q or H and x3 is T or H
<220>
   <221> X1
   <222> (33)..(33)
   <223> X1 is L or H
<220>
   <221> X2
   <222> (89)..(89)
   <223> X2 is Q or H
<220>
   <221> X3
   <222> (97)..(97)
   <223> x3 is T or H
<400> 24
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn His
               20                  25                  30
   Xaa Ala Trp Tyr Gln Gln Lys Pro Gly Lys Ala Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Xaa His Tyr His Arg Ala Pro Tyr
                   85                  90                  95
   Xaa Phe Gly Gln Gly Thr Lys Val Glu Ile Lys
               100                 105
<210> 25
   <211> 121
   <212> PRT
   <213> Artificial sequence
<220>
   <223> histidine-mutated adalimumab heavy chain variable region
<400> 25
   Glu Val Gln Leu Val Glu Ser Gly Gly Gly Leu Val Gln Pro Gly Arg
   1 5 10 15
   Ser Leu Arg Leu Ser Cys Ala Ala Ser Gly Phe Thr Phe Asp Asp Tyr
   20 25 30
   Ala Met His Trp Val Arg Gln Ala Pro Gly Lys Gly Leu Glu Trp Val
   35 40 45
   Ser Ala Ile Thr Trp Asn Ser Gly His Ile Asp Tyr Ala Asp Ser Val
   50 55 60
   Glu Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Lys Val Ser Tyr His Ser Thr Ala Ser Ser Leu Asp Tyr Trp Gly
   100 105 110
   Gln Gly Thr Leu Val Thr Val Ser Ser
   115 120
<210> 26
   <211> 121
   <212> PRT
   <213> Artificial Sequence
<220>
   <223> histidine-mutated adalimumab comprising the variable heavy chain sequence
<400> 26
   Glu Val Gln Leu Val Glu Ser Gly Gly Gly Leu Val Gln Pro Gly Arg
   1 5 10 15
   Ser Leu Arg Leu Ser Cys Ala Ala Ser Gly Phe Thr Phe Asp Asp Tyr
   20 25 30
   Ala Met His Trp Val Arg Gln Ala Pro Gly Lys Gly Leu Glu Trp Val
   35 40 45
   Ser Ala Ile Thr Trp Asn Ser Gly His Ile Asp Tyr Ala Asp Ser Val
   50 55 60
   Glu Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Lys Val Ser Tyr Leu Ser Thr Ala His His Leu Asp Tyr Trp Gly
   100 105 110
   Gln Gly Thr Leu Val Thr Val Ser Ser
   115 120
<210> 27
   <211> 107
   <212> PRT
   <213> Artificial sequence
<220>
   <223> histidine-mutated adalimumab comprising the variable heavy chain sequence
<400> 27
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn His
               20                  25                  30
   His Ala Trp Tyr Gln Gln Lys Pro Gly Lys Ala Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asn Arg Ala Pro Tyr
                   85                  90                  95
   Thr Phe Gly Gln Gly Thr Lys Val Glu Ile Lys
               100                 105
<210> 28
   <211> 107
   <212> PRT
   <213> Artificial sequence
<220>
   <223> histidine-mutated adalimumab variable light chain sequence
<400> 28
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys His His Tyr His Arg Ala Pro Tyr
                   85                  90                  95
   Thr Phe Gly Gln Gly Thr Lys Val Glu Ile Lys
               100                 105
<210> 29
   <211> 107
   <212> PRT
   <213> Artificial Sequence
<220>
   <223> histidine-mutated adalimumab variable light chain
<400> 29
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln His Tyr His Arg Ala Pro Tyr
                   85                  90                  95
   His Phe Gly Gln Gly Thr Lys Val Glu Ile Lys
               100                 105
<210> 30
   <211> 107
   <212> PRT
   <213> Artificial Sequence
<220>
   <223> histidine-mutated adalimumab variable light chain
<400> 30
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Arg Asn Tyr
   20 25 30
   Leu Ala Trp Tyr Gln Gln Lys Pro Gly Lys Ala Pro Lys Leu Leu Ile
   35 40 45
   Tyr Ala Ala Ser Thr Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Val Ala Thr Tyr Tyr Cys Gln His Tyr His Arg Ala Pro Tyr
   85 90 95
   His Phe Gly Gln Gly Thr Lys Val Glu Ile Lys
   100 105
<210> 31
   <211> 12
   <212> PRT
   <213> Artificial sequence
<220>
   <223> mutated adalimumab CDR3 heavy chain
<400> 31
   Val His Tyr His Ser Thr Ala Ser Ser Leu Asp Tyr
   1 5 10
<210> 32
   <211> 7
   <212> PRT
   <213> Artificial Sequence
<220>
   <223> mutated adalimumab CDR2 light chain
<400> 32
   Ala Ala His Thr Leu Gln Ser
   1 5
<210> 33
   <211> 107
   <212> PRT
   <213> Artificial Sequence
<220>
   <223> histidine-mutated adalimumab light chain variable region
<400> 33
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln His Tyr His Arg Ala Pro Tyr
                   85                  90                  95
   Thr Phe Gly Gln Gly Thr Lys Val Glu Ile Lys
               100                 105
<210> 34
   <211> 107
   <212> PRT
   <213> Artificial sequence
<220>
   <223> histidine-mutated adalimumab light chain variable region
<400> 34
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn His
               20                  25                  30
   Leu Ala Trp Tyr Gln Gln Lys Pro Gly Lys Ala Pro Lys Leu Leu Ile
           35                  40                  45
   Tyr Ala Ala Ser Thr Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Val Ala Thr Tyr Tyr Cys Gln Arg Tyr Asn His Ala Pro Tyr
   85 90 95
   Thr Phe Gly Gln Gly Thr Lys Val Glu Ile Lys
   100 105
<210> 35
   <211> 107
   <212> PRT
   <213> Artificial sequence
<220>
   <223> histidine-mutated adalimumab light chain variable region
<400> 35
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Arg Asn His
   20 25 30
   Leu Ala Trp Tyr Gln Gln Lys Pro Gly Lys Ala Pro Lys Leu Leu Ile
   35 40 45
   Tyr Ala Ala Ser Thr Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Val Ala Thr Tyr Tyr Cys Gln Arg Tyr Asn Arg Ala Pro Tyr
   85 90 95
   Thr Phe Gly Gln Gly Thr Lys Val Glu Ile Lys
   100 105
<210> 36
   <211> 107
   <212> PRT
   <213> Artificial Sequence
<220>
   <223> histidine-mutated adalimumab light chain variable region
<400> 36
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Arg Asn Tyr
   20 25 30
   Leu Ala Trp Tyr Gln Gln Lys Pro Gly Lys Ala Pro Lys Leu Leu Ile
   35 40 45
   Tyr Ala Ala Ser Thr Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Val Ala Thr Tyr Tyr Cys Gln Arg His Asn Arg Ala Pro Tyr
   85 90 95
   Thr Phe Gly Gln Gly Thr Lys Val Glu Ile Lys
   100 105
<210> 37
   <211> 107
   <212> PRT
   <213> Artificial Sequence
<220>
   <223> histidine-mutated adalimumab light chain variable region
<400> 37
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Arg Asn His
   20 25 30
   Leu Ala Trp Tyr Gln Gln Lys Pro Gly Lys Ala Pro Lys Leu Leu Ile
   35 40 45
   Tyr Ala Ala His Thr Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Val Ala Thr Tyr Tyr Cys Gln Arg Tyr Asn Arg Ala Pro Tyr
   85 90 95
   Thr Phe Gly Gln Gly Thr Lys Val Glu Ile Lys
   100 105
<210> 38
   <211> 9
   <212> PRT
   <213> Artificial sequence
<220>
   <223> mutated adalimumab CDR3 light chain
<400> 38
   Gln Arg His Asn Arg Ala Pro Tyr Thr
   1               5
<210> 39
   <211> 121
   <212> PRT
   <213> Artificial Sequence
<220>
   <223> mutated adalimumab variable heavy chain
<400> 39
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val His Tyr His Ser Thr Ala Ser Ser Leu Asp Tyr Trp Gly
               100                 105                 110
   Gln Gly Thr Leu Val Thr Val Ser Ser
           115                 120
<210> 40
   <211> 12
   <212> PRT
   <213> Artificial sequence
<220>
   <223> mutated adalimumab CDR3 heavy chain, wherein X1 is S or H; X2 is L or H, X3 is S or H, and X4 is S or H, and wherein at least X1 or x2 or X3 or x4 is H
<220>
   <221> X1
   <222> (2)..(2)
   <223> X1 is S or H
<220>
   <221> X2
   <222> (4)..(4)
   <223> X2 is L or H
<220>
   <221> X3
   <222> (8)..(8)
   <223> X3 is S or H
<220>
   <221> X4
   <222> (9)..(9)
   <223> X4 is S or H
<400> 40
   Val Xaa Tyr Xaa Ser Thr Ala Xaa Xaa Leu Asp Tyr
   1               5                   10
<210> 41
   <211> 9
   <212> PRT
   <213> Artificial sequence
<220>
   <223> mutated adalimumab CDR3 light chain, wherein X1 is Q or H, x2 is R or H, X3 is Y or H, X4 is N or H, and X5 is T or H, wherein at least X1 or x2 or X3 or x4 or X5 is H
<220>
   <221> X1
   <222> (1)..(1)
   <223> X1 is Q or H
<220>
   <221> X2
   <222> (2)..(2)
   <223> X2 is R or H
<220>
   <221> X3
   <222> (3)..(3)
   <223> X3 is Y or H
<220>
   <221> X4
   <222> (4)..(4)
   <223> X4 is N or H
<220>
   <221> X5
   <222> (9)..(9)
   <223> X5 is T or H
<400> 41
   Xaa Xaa Xaa Xaa Arg Ala Pro Tyr Xaa
   1               5
<210> 42
   <211> 11
   <212> PRT
   <213> Artificial Sequence
<220>
   <223> mutated adalimumab CDR1 light chain , wherein X1 is Y or H, x2 is L or H, wherein at least x1 or X2 is H.
<220>
   <221> X1
   <222> (9)..(9)
   <223> X1 is Y or H
<220>
   <221> X2
   <222> (10)..(10)
   <223> X2 is L or H
<400> 42
   Arg Ala Ser Gln Gly Ile Arg Asn Xaa Xaa Ala
   1               5                   10
<210> 43
   <211> 107
   <212> PRT
   <213> Artificial Sequence
<220>
   <223> mutated adalimumab light chain variable region, wherein X1 is Y or H, X2 is L or H, x3 is S or H, x4 is Q or H, x5 is R or H, X6 is Y or H, X7 is N or H, x8 is T or H, wherein at least x1 or x2 or X3 or X4 or X5 or x6 or X7 or x8 is H.
<220>
   <221> X1
   <222> (32)..(32)
   <223> X1 is Y or H
<220>
   <221> X2
   <222> (33)..(33)
   <223> X2 is L or H
<220>
   <221> X3
   <222> (52)..(52)
   <223> X3 is S or H
<220>
   <221> X4
   <222> (89)..(89)
   <223> X4 is Q or H
<220>
   <221> X5
   <222> (90)..(90)
   <223> X5 is R or H
<220>
   <221> X6
   <222> (91)..(91)
   <223> X6 is Y or H
<220>
   <221> X7
   <222> (92)..(92)
   <223> X7 is N or H
<220>
   <221> X8
   <222> (97)..(97)
   <223> X8 is T or H
<400> 43
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Xaa
               20                  25                  30
   Xaa Ala Trp Tyr Gln Gln Lys Pro Gly Lys Ala Pro Lys Leu Leu Ile
           35                  40                  45
   Tyr Ala Ala Xaa Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Xaa Xaa Xaa Xaa Arg Ala Pro Tyr
                   85                  90                  95
   Xaa Phe Gly Gln Gly Thr Lys Val Glu Ile Lys
               100                 105
<210> 44
   <211> 121
   <212> PRT
   <213> Artificial sequence
<220>
   <223> mutated adalimumab heavy chain variable region, wherein X1 is S or H, x2 is L or H, X3 is s or H, X4 is S or H, wherein at least X1 or X2 or X3 or X4 is H.
<220>
   <221> X1
   <222> (100)..(100)
   <223> X1 is S or H
<220>
   <221> X2
   <222> (102)..(102)
   <223> X2 is L or H
<220>
   <221> x3
   <222> (106)..(106)
   <223> X3 is S or H
<220>
   <221> X4
   <222> (107)..(107)
   <223> X4 is S or H
<400> 44
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Xaa Tyr Xaa Ser Thr Ala Xaa Xaa Leu Asp Tyr Trp Gly
               100                 105                 110
   Gln Gly Thr Leu Val Thr Val Ser Ser
           115                 120